# EUROPEAN PATENT APPLICATION

(11) **EP 1 561 470 A1**
(43) Date of publication of application: **10.08.2005**
(21) Application number: 04102774.9
(22) Date of filing: 17.06.2004
(51) Int. Cl.: A61K 39/29, A61P 31/14

(54) **HCV vaccines comprising the envelope-1 (E1) protein or the corresponding DNA and at least one antiviral agent**

(30) Priority: 20.06.2003 EP 03447161; 20.06.2003 US 479844 P
(71) Applicant: INNOGENETICS N.V., 9052 Gent (BE)
(72) Inventor: Maertens, Geert, 8310 Brugge (BE); Depla, Erik, 9070 Destelbergen (BE); Vander Stichele, Christine, 9000 Ghent (BE); Hulstaert, Frank, 9090 Melle (BE)

(57) **Abstract**

The invention relates to therapy of HCV infection, and more particularly to combination therapy of HCV infection. The combination therapy comprises combination of a HCV E1 vaccine and an antiviral agent. In one aspect, the antiviral agent may be an interferon.

## Description

### FIELD OF THE INVENTION

The invention relates to the field of therapy of HCV infection, and more particularly to combination therapy of HCV infection. The combination therapy comprises combination of an HCV vaccine and an antiviral agent.

### BACKGROUND OF THE INVENTION

The about 9.6 kb single-stranded RNA genome of the HCV virus comprises a 5'- and 3'-non-coding region (NCRs) and, in between these NCRs a single long open reading frame of about 9 kb encoding an HCV polyprotein of about 3000 amino acids.

HCV polypeptides are produced by translation from the open reading frame and cotranslational proteolytic processing. Structural proteins are derived from the amino-terminal one-fourth of the coding region and include the capsid or Core protein (about 21 kDa), the E1 envelope glycoprotein (about 35 kDa) and the E2 envelope glycoprotein (about 70 kDa, previously called NS1), and p7 (about 7kDa). The E2 protein can occur with or without a C-terminal fusion of the p7 protein (Shimotohno et al. 1995). Recently, an alternative open reading frame in the Core-region was found which is encoding and expressing a protein of about 17 kDa called F (Frameshift) protein (Xu et al. 2001; Ou & Xu in US Patent Application Publication No. US2002/0076415). In the same region, ORFs for other 14-17 kDa ARFPs (Alternative Reading Frame Proteins), A1 to A4, were discovered and antibodies to at least A1, A2 and A3 were detected in sera of chronically infected patients (Walewski et al. 2001). From the remainder of the HCV coding region, the non-structural HCV proteins are derived which include NS2 (about 23 kDa), NS3 (about 70 kDa), NS4A (about 8 kDa), NS4B (about 27 kDa), NS5A (about 58 kDa) and NS5B (about 68 kDa) (Grakoui et al. 1993).

HCV is the major cause of non-A, non-B hepatitis worldwide. Acute infection with HCV (20% of all acute hepatitis infections) frequently leads to chronic hepatitis (70% of all chronic hepatitis cases) and end-stage cirrhosis. It is estimated that up to 20% of HCV chronic carriers may develop cirrhosis over a time period of about 20 years and that of those with cirrhosis between 1 to 4%/year is at risk to develop liver carcinoma. (Lauer & Walker 2001, Shiffman 1999). An option to increase the life-span of HCV-caused end-stage liver disease is liver transplantation (30% of all liver transplantations world-wide are due to HCV-infection).

HCV-monotherapies based on interferon (IFN) which are currently FDA-approved or which are being tested in (pre)clinical trials, include therapies with natural IFN-alfa or its pegylated form, with IFN-alfacon-1, lymphoblastoid IFN-alfa-n1, IFN-alfa-2a or its pegylated form, IFN-alfa-2b or its pegylated form, a fusion protein between albumin and IFN-alfa-2b, IFN-beta-1a, and IFN-omega (Tan et al. 2002).

Combination therapies involving IFN which are currently FDA-approved or which are being tested in (pre)clinical trials include therapies with IFN-alfa-2b and ribavirin, pegylated IFN-alfa-2b and ribavirin, pegylated IFN-alfa-2a and ribavirin, IFN-alfa-2b and thymosin-alfa-1, pegylated IFN-alfa-2a with histamine dihydrochloride, and IFN-beta with the IFN-enhancer EMZ701 (Transition Therapeutics) (Tan et al. 2002).

The approved options for treating HCV infection are very limited and normally comprise a treatment regimen of the ribavirin and interferon-alfa (or pegylated interferon-alfa). The current state of the art is that IFN-alfa contributes a pronounced antiviral effect. The mechanism contributing to the synergistic effect of ribavirin with interferons remains unresolved (e.g. ribavirin has no antiviral effect as a monotherapy). Prolonged administration of relative high amounts of interferon, whether alone or in combination with ribavirin, may cause the induction of autoimmune disorders. This combination therapy displays severe side effects on the cardiovascular system, with neutropenia caused by interferon. Ribavirin is known to induce hemolysis resulting in anemia. Interferon is also notorious for its inductive or worsening effect on depression in patients treated therewith (Chutaputti 2000). Even the most optimal treatment regimen today (combination of pegylated interferon-alfa with ribavirin and with extension of the therapy based on genotype and viral load) results in severe side effects (about 10% of patients have to discontinue because of side effects, and overall about 25% of patients stop therapy prematurely), and of those able to complete the treatment schedule only 42-46% show a sustained response if they are infected with genotype 1, the most predominant genotype world-wide (Manns et al. 2001). In addition, this therapy is not advised for patients with pre-existing markers of anemia, auto-immune diseases or a history of depression which are already frequent conditions in HCV. Because of these and other medical complications, up to 75% of the HCV patients are excluded from therapy today (Falck-Ytter et al. 2002). Schering-Plough calculated the number of people who have not responded to the current therapies to increase to 1 million by 2010 (J. Albrecht, Schering-Plough satellite symposium, EASL, Madrid, April 2002).

Many drugs targeting different HCV proteins are currently being developed. Some of these, such as a number of HCV RNA, HCV internal ribosome entry site (IRES), HCV NS3 and HCV NS5B inhibitors are currently in preclinical phase or in different clinical phases (see, e.g., Tan et al. 2002).

Another approach is based on the use of vaccines. An HCV vaccine may be a DNA-based vaccine, a protein- or peptide-based vaccine, or a combination of a prime and a boost vaccination may be applied. Both prime or booster vaccines may be comprised of DNA, vector, protein, or peptide components. Since DNA and vector-based vaccines result in the production of protein in vivo, mainly vaccinations involving HCV proteins or peptides are discussed hereafter.

In mice, DNA-prime protein-boost vaccination studies have been performed using Core (Hu et al. 1999) and E2 (Song et al. 2000). Core DNA vaccination produced a predominantly IgM antibody production whereas protein boosting caused an increase in IgG antibody levels. The T-cell proliferation response induced by Core DNA-vaccination was increased by the protein boost. A CTL response was not observed when the Core protein alone was injected but was detectable both in DNA-primed and in DNA-primed protein-boosted vaccinations. For E2, both the antibody response and the CTL response elicited upon DNA vaccination were augmented by protein boosting. Studies with protein-based HCV vaccines are very limited and include immunization of mice with fragments of Core (Shirai et al. 1996, Hu et al. 1999), E1 (Lopez-Diaz de Cerio et al. 1999), E2 (Nakano et al. in US Patent Publication No. 2002/0119495; Houghton et al. in US Patent Application Publication No. 2002/0002272), E1/E2 or E1/E2 + Core (Drane et al. in International Patent Publication No. WO01/37869) and NS5 (Shirai et al. 1996, Uno-Furuta et al. 2001).

Until today, studies in primates or humans remain very scarce, yet these are of considerably higher interest. More particularly, prophylactic and therapeutic vaccinations performed in chimpanzees or therapeutic vaccinations of HCV-infected humans can yield results which are very different from those obtained in mice, especially with DNA-based vaccines. E2 DNA-vaccinations of mice, macaques and chimpanzees were described in two studies of Forns et al. (1999, 2000). Rhesus macaques were injected with Core-expressing vaccinia virus, Core adjuvanted with LTK63 or Core adjuvanted with ISCOM in a study by Drane et al. (in International Patent Publication No. WO01/37869).

Prophylactic vaccination of chimpanzees with an E1/E2 or Core/E1/E2 complex has been described in Choo et al. (1994), Houghton et al. (1995). Prophylactic and therapeutic vaccination of chimpanzees with an E1 protein has been described in WO99/67285 and WO02/055548. Interestingly, the immune responses to E1 observed in chimpanzees were also observed in HCV-infected humans and in healthy volunteers. The E1-subunit vaccine is currently in phase II clinical trials (WO02/05548; Nevens et al. 2003).

US 2002/0155124 (WO02/13855) discloses vaccine compositions comprising ribavirin. Recombinant HCV NS3 is an exemplified antigen to which humoral and cellular immune responses may be increased by means of local co-administration of ribavirin as adjuvant.

WO02/22155 discloses a composition comprising HCV E2 lacking the HVR1 region. Said composition may further comprise an immunomodulatory agent such as interferon-gamma. Also disclosed is a method of treating a HCV infection therewith.

There is a clear need to improve existing therapies of HCV infection as well as to explore other types of therapy.

### SUMMARY OF THE INVENTION

A first aspect of the invention relates to the use of an isolated HCV E1 protein and/or of an HCV E1 DNA vector for the manufacture of an HCV vaccine or an HCV immunogenic composition
- for therapeutic treatment of an HCV-infected mammal; and/or
- for inducing an immune response in an HCV-infected mammal; and/or
- for enhancing HCV viral clearance in an HCV-infected mammal; and/or
- for suppressing HCV viral breakthrough in an HCV-infected mammal; and/or
- for suppressing HCV viral rebound after cessation of therapeutic treatment of an HCV-infected mammal
by a method comprising administering said HCV vaccine before and/or during and/or after a therapy comprising or with at least one antiviral agent.

Alternatively, the use of at least one antiviral agent for the manufacture of a pharmaceutical composition
- for therapeutic treatment of an HCV-infected mammal; and/or
- for inducing an immune response in an HCV-infected mammal; and/or
- for enhancing HCV viral clearance in an HCV-infected mammal; and/or
- for suppressing HCV viral breakthrough in an HCV-infected mammal; and/or
- for suppressing HCV viral rebound after cessation of therapeutic treatment of an HCV-infected mammal
by a method comprising administering said pharmaceutical composition before and/or during and/or after a therapy comprising administering an HCV vaccine or an HCV immunogenic composition wherein said HCV vaccine or HCV immunogenic composition is at least comprising an isolated HCV E1 protein and/or of an HCV E1 DNA vector as active substance.

In specific embodiments to any of the recited uses said at least one antiviral agent may be an interferon or a variant or consensus sequence thereof, or an inducer of interferon. Said interferon may be a type I or a type III interferon or a variant or consensus sequence of any thereof. In particular, said type I interferon is chosen from an interferon-alfa, interferon-beta, interferon-omega or interferon-tau or said type III interferon is chosen from IL-28A, IL-28B or IL-29. Said inducer of interferon may be chosen from TLR7-ligands, TLR-9 ligands, ANA245, or ANA971.

Another embodiment relates to the at least one antiviral agent that may be chosen from an IMPDH-inhibitor, an anti-HCV antibody, an immune modulator, a HCV-receptor inhibitor, an HCV fusion inhibitor, a modulator of host cell function required for HCV replication, a molecule targeting a step in the HCV life cycle, a molecule targeting an HCV IRES, a molecule binding to HCV RNA, a molecule targeting an HCV NS2-NS3 protease, a molecule targeting an HCV NS3 protease, a molecule targeting an HCV NS3 RNA helicase, a molecule targeting an HCV NS3-NS4A protease, a molecule targeting an HCV NS5B RNA-dependent RNA polymerase, and a molecule inhibiting binding of HCV E2 or HCV NS5 to protein kinase R, a molecule targeting an HCV Core protein, a molecule targeting an HCV E1 protein or a molecule targeting an HCV E2 protein

In a further aspect of the invention and if said at least one antiviral agent in the uses recited in the first aspect of the invention is interferon or a variant or consensus sequence thereof, the methods of said uses may further comprise a therapy comprising or with an interferon-enhancer or at least one additional antiviral agent.

In a specific embodiment thereto, said interferon-enhancer is chosen from interferon gamma, ribavirin, levovirin, viramidine, amantadine, thymosin alfa-1, histamine dihydrochloride, a methyldonor, or ANA246.

In another specific embodiment, said at least one additional antiviral agent is chosen from an IMPDH-inhibitor, an anti-HCV antibody, an immune modulator, a HCV-receptor inhibitor, an HCV fusion inhibitor, a modulator of host cell function required for HCV replication, a molecule targeting a step in the HCV life cycle, a molecule targeting an HCV IRES, a molecule binding to HCV RNA, a molecule targeting an HCV NS2-NS3 protease, a molecule targeting an HCV NS3 protease, a molecule targeting an HCV NS3 RNA helicase, a molecule targeting an HCV NS3-NS4A protease, a molecule targeting an HCV NS5B RNA-dependent RNA polymerase, and a molecule inhibiting binding of HCV E2 or HCV NS5 to protein kinase R, a molecule targeting an HCV Core protein, a molecule targeting an HCV E1 protein or a molecule targeting an HCV E2 protein.

Said IMPDH-inhibitor may be chosen from ribavirin or a variant thereof, viramidine, mizoribine monophosphate, merimepodib or mycophenolic acid or a variant thereof.

If the interferon-enhancer or IMPDH-inhibitor applied in any of the above uses is ribavirin or a variant thereof, then administration of an ameliorant of ribavirin-induced anemia to the HCV-infected mammal is a further aspect of the invention. Said ameliorant of ribavirin-induced anemia may be an antioxidant or erythropoietin.

Another aspect of the invention covers a pharmaceutical pack or kit at least comprising one or more containers with an HCV vaccine and one or more containers with at least one antiviral agent and wherein said HCV vaccine is at least comprising an isolated HCV E1 protein and/or an HCV E1 DNA vector as active substance.

In one embodiment, said at least one antiviral agent is an interferon or a variant or consensus sequence thereof or an inducer of interferon.

In a specific embodiment to the uses of the invention and the pharmaceutical packs or kits of the invention, the HCV E1 protein may be an E1s protein or the HCV E1 DNA vector may be encoding an E1s protein. Said HCV E1 protein may be the HCV E1 protein defined by SEQ ID NO:1 or said HCV E1 DNA vector may be encoding the HCV E1 protein defined by SEQ ID NO:1.

In another specific embodiment to the uses of the invention and the pharmaceutical packs or kits of the invention said HCV E1 protein may be added to said HCV vaccine as a viral-like particle.

In a further specific embodiment to the uses of the invention and the pharmaceutical packs or kits of the invention the cysteine-thiol groups in said HCV E1 protein may be reversibly or irreversibly blocked.

### FIGURE LEGENDS

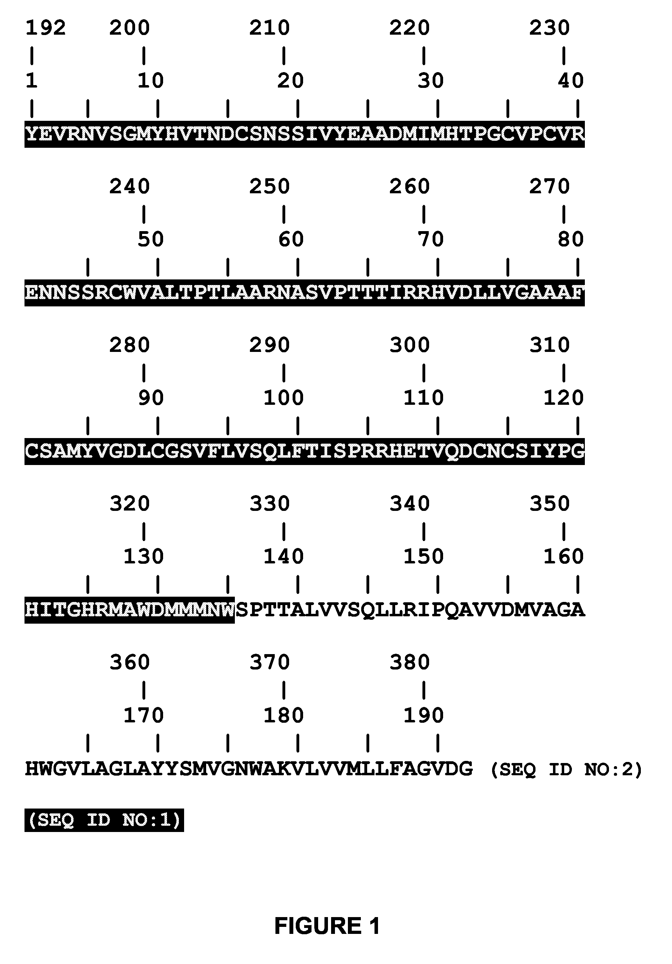

**Figure 1.** Amino acid sequence of a HCV type 1b E1 protein wherein the amino acids are numbered 1-192 and, according to the position in the HCV polyprotein, 192-383 (SEQ ID NO:2). The E1s part of this protein is indicated in a black shaded box (SEQ ID NO:1).

### DETAILED DESCRIPTION OF THE INVENTION

### General definitions

An "antiviral agent or compound" is defined as an agent which, when administered to a patient, is capable of significantly reducing the titer of viral nucleic acids (HCV RNA in the case of HCV infection) in the blood or serum either directly (e.g., by inhibiting a viral enzyme activity) or indirectly (e.g., via modulation of the antiviral responses of a host cell), either transiently or in a sustained way. In general, the term antiviral agents as used herein comprise any pharmaceutically acceptable form of said agents including pharmaceutically acceptable salts and solvents as long as the biological effectiveness of the antiviral agent is not significantly compromised. Many solvents exist and the choice of the solvent will depend on possible other components of the composition in which the antiviral agent is present. Exemplary solvents include water, methanol, ethanol, isopropanol, DMSO, acetic acid. Prodrugs or precursor compounds of antiviral agents are also included as long as in vivo conversion or conversion under physiological conditions of the prodrug or precursur to the antiviral agents is ensured. Variants of an antiviral agent or prodrugs of an antiviral agent are usually employed to remedy problems of stability, toxicity or bioavailability.

A "combination therapy" comprises administering at least two different antiviral drugs or compounds to a patient as treatment of a given disease; this in contrast to a monotherapy which comprises administering a single antiviral drug or compound to a patient. The IFN-ribavirin combination therapy as treatment of chronic HCV infection involves subcutaneous administration of IFN and oral (or systemic) administration of ribavirin in the form of capsules or tablets. The different drugs of a combination therapy may thus be administered via different routes.

"Viral load or viremia level" is the amount of viral nucleic acids present in the blood or serum. The viremia level is the amount of HCV RNA per ml of serum or blood, or per gram of tissue.

"Viral breakthrough" is meant to define the reappearance of detectable viral nucleic acids in the blood or serum during a therapeutic treatment or therapy, whether monotherapy or combination therapy. Such a viral breakthrough usually appears during such treatment that initially appears to be successful based on the disappearance of detectable viral nucleic acids.

A "sustained virological response or SVR" is a sustained lack of detectable viral nucleic acids (HCV RNA in the case of HCV infection) in the blood or serum six months after cessation or completion of a therapeutic treatment or therapy (i.e. after end-of-treatment or EOT), whether monotherapy or combination therapy.

In contrast thereto, "viral rebound or relapse" is the re-emergence of detectable viral nucleic acids (HCV RNA in the case of HCV infection) in the blood or serum within six months after cessation or completion of a successful therapeutic treatment or therapy, whether monotherapy or combination therapy.

It is possible that currently undetectable levels of HCV RNA (e.g. such as those which must be present in an on-treatment responder who later shows a breakthrough or relapse) become detectable in the future due to the use of detection methods with increased sensitivity. In such case the responses will be defined in other ways that may be defined in the future.

A "biochemical response" is the decrease to normal values of detectable levels of liver enzyme activity in the blood or serum of an HCV-infected mammal as a result of a therapeutic treatment or therapy. Liver enzymes include alanine aminotransferase (ALT) and gamma-glutamylpeptidase (GGT).

The HCV viral life cycle is meant to comprise all steps including the process of receptor binding, conformational change of the envelope, cell entry, membrane fusion, uncoating,translation (initiation) of the HCV polyprotein, proteolytic processing of the nascent HCV polyprotein, replication of the HCV viral genome, the processes of viral assembly and maturation until the process of budding and secretion of the newly formed HCV viral particles. Also included are processes and interactions of regulation by HCV RNAs or proteins, comprising but not limited to immunomodulation, inhibition of innate and adaptive immune defence mechanisms, regulation of the cell cycle, and regulation of the oxidative stress processes.

The terms protein, polypeptide and peptide are used interchangeably herein.
The term "immunogenic" refers to the ability of a protein (whether or not as product of expression from a DNA vaccine vector) or a substance to produce at least one element of an immune response. The immune response is the total response of the body of an animal to the introduction of an antigen and comprises multiple elements including antibody formation (humoral response or humoral immunity), cellular immunity, hypersensitivity, or immunological tolerance. Cellular immunity refers to cellular responses elicited by an antigen and include a T-helper cell- and/or CTL-response and/or stimulated cytokine production. The term "antigen" refers to the ability of a peptide, protein or other substance to be antigenic or immunogenic. An antigen is understood to comprise at least one epitope.

"Antigenic" refers to the capability of a protein (whether or not as product of expression from a DNA vaccine vector) or substance to be recognized by an elicited humoral and/or cellular immune response. Typically, the antigenic quality of a protein or substance is determined by in vitro assays. For humoral responses, a protein or substance can be referred to as antigenic in case the protein or substance is recognized by elicited antibodies in e.g. an ELISA, western-blot, RIA, immunoprecipitation assay or any similar assay in which the protein or substance is allowed to be recognized by an elicited antibody and in which such a recognition can be measured by, e.g., a colorometric, fluorometric or radioactive detection, or formation of a precipitate. For cellular response, a protein or substance can be referred to as antigenic in case the protein or substance is recognized by an elicited T-cell response in e.g. an T-cell proliferation assay, a ⁵¹Cr-release assay, a cytokine secretion assay or alike in which the protein or substance is incubated in the presence of T-cells drawn from an individual in which immune response have been elicited and in which a recognition by the T-cell is measured by, e.g., a proliferative response, a cell lysis response, a cytokine secretion. An antigenic protein or substance may be immunogenic in se but may also require additional structures to be rendered immunogenic.
An "epitope" refers to a structure capable of binding to and/or activating a cell involved in eliciting an immune response to said structure. Epitopes thus include epitopes of B-cells, T-cells, T-helper cells and CTLs. Epitopes include conformational epitopes and linear epitopes. A linear epitope is a limited set of, e.g., contiguous elements of a repetitive structure construed with a limited number of distinct elements. A conformational epitope usually comprises, e.g., discontigous elements of such a repetitive structure which are, however, in close vicinity due to the three-dimensional folding of said repetitive structure. A well-known example of such a repetitive structure is a peptide or protein wherein the contiguous or discontiguous elements are amino acids. Peptide- or protein-epitopes comprise peptides or parts of peptides or proteins capable of binding to, e.g., T-cell receptors, B-cell receptors, antibodies or MHC molecules. The size of linear peptide- or protein-epitopes can be limited to a few, e.g. 4, 5, 6, 7, 8, 9, 10, 11, 12, 13, 14 or 15 amino acids. An epitope is antigenic but not always immunogenic.

A T-cell stimulating epitope refers to an epitope capable of stimulating T-cells, T-helper cells or CTL-cells. A T-helper cell stimulating epitope may be selected by monitoring the lymphoproliferative response, also referred to as CD4⁺ T-cell proliferation response, towards (potential antigenic) polypeptides containing in their amino acid sequence a (putative) T-cell stimulating epitope. Said lymphoproliferative response may be measured by either a T-helper assay comprising *in vitro* stimulation of peripheral blood mononuclear cells (PBMCs) from patient sera with varying concentrations of peptides to be tested for T-cell stimulating activity and counting the amount of radiolabelled thymidine taken up by the PBMCs. Proliferation is considered positive when the stimulation index (mean cpm of antigen-stimulated cultures/mean cpm of controle cultures) is more than 1, preferably more than 2, most preferably more than 3. A CTL-stimulating epitope may be selected by means of a cytotoxic T-lymphocyte or cytotoxic T-cell (CTL) assay measuring the lytic activity of cytotoxic cells, also referred to as CD8⁺ CTL response, using ⁵¹Cr release. Cell-mediated responses may also be assessed by measuring cytokine production, e.g., by an ELISpot assay (see for instance Fujihashi et al. 1993). Characteristic for a Th1-like response is the production/secretion of, e.g., IL-2 and/or IFN-γ. Characteristic for a Th2-like response is the production/secretion of, e.g., IL-4 and/or IL-5 and/or IL-10.

### Antiviral compounds or agents

The main human interferons are IFN-alfa, IFN-beta and IFN-gamma. Type 1 interferons include IFN-alfa, IFN-beta, IFN-delta, IFN-omega, and IFN-tau. IFN-gamma is a type 2 interferon. Type 3 interferons include IL-28A, IL-28B or IL-29 and are distantly related to type 1 interferons. Type 3 interferons may thus serve as alternative of type 1 interferons in antiviral therapy (Sheppard et al. 2003, Vilcek 2003).

Induced gene expression by binding of an interferon to its receptor leads to expression of proteins thought to be (partly) responsible for the biological activities of the interferons. Interferons are known for their wide spectrum antiviral activities. The type 1 interferons alfa, - beta, -omega and -tau or variants or consensus sequences of any thereof, are currently used in (or tested for) treatment of HCV infection.

An interferon variant may be any variant of interferon still displaying its biological activity. Variants include conjugates with other molecules such as sugars or polymers (e.g., polyethyleneglycol = PEG), fusion proteins with other proteins such as albumin, multimers of the same interferon, hybrids of different interferons, muteins such as amino acid substitution or deletion variants (e.g., interferon with altered cysteine pattern), analogues (e.g., with reduced toxicity), and splice variants. Many of these variants have been described for different interferon types and disclosures include US 5,071,761, US 6,300,475, US 6,300,474, US,6,204,022,US,5,981,709,US,5,951,974 ,US 5,723,121 , US 4,820,638, US 4,780,530, US 6,572,853, US 6,531,122, US 6,514,729, US 2002/0169290, US 2002/0155547, US 5,723,121, US 4,914,033, US 4,793,995, US 4,753,795, US 4,751,077, US 4,738,845, US 4,738,844, US 2002/0192183, US 6,497,871, US 6,120,762, US 6,046,034, US 5,690,925, US 4,898,931, US 4,845,196, US 4,835,256, US 4,917,887, US 4,892,743, US 4,885,166, US 4,758,428, US 4,751,077, US 4,588,585, US 6,174,996, US 5,939,286, EP 1 156 771, EP 0 170 204, EP 0 170 204, WO 03/016472, WO 00/78266, JP 11042089, JP 10295382, and JP 10113191. "Consensus interferons or CIFN" have been disclosed in, e.g., US 4,897,471.

A subset of HCV genotype 1b-infected patients not responding to a first administration of IFN-alfa was reported to be convertible to IFN-alfa responders when these patients were treated with IFN-gamma prior to or concurrent with further IFN-alfa treatment (US 2003/0003075 and US 6,455,051). IFN-gamma may thus be used as an enhancer of IFN-alfa.

Another enhancer of interferon activity is, e.g., a polymer of 3-oxygermylpropionic acid as disclosed in EP 0 652 223. Methyldonor-compounds such as folic acid or vitamin B12 as enhancers of interferon activity are disclosed in, e.g., WO 03/030929. IFN effects are also enhanced by amantadine, thymosin alfa-1, histamine dihydrochloride and interleukin-12 (IL-12).

Several compounds are known to induce IFN, these compounds include nucleoside analogs such as ANA-245 and ANA-971. Ligands of the toll-like receptors TLR7 and TLR9 are further examples of interferon-inducing compounds (Ito et al. 2002). TLR7 ligands include imidazoquinolinamines such as imiquimod and resiquimod (Dockrell and Kinghorn 2001, Hemmi et al. 2002) and certain guanine ribonucleosides such as C8-substituted or N7, C8-disubstituted guanine ribonucleosides (Lee et al. 2003).

The action of ribavirin (1-β-D-ribofuranosyl-1,2,4-triazole-3-carboxamide) or ribavirin-triphosphate (1-β-D-ribofuranosyl-1,2,4-triazole-3-carboxamide-5'-triphosphate) on HCV is unclear. Although ribavirin monotherapy is capable of reducing serum alanine aminotransferase (ALT) levels in chronic HCV patients, there is no effect on levels of HCV-RNA, i.e., replication of the virus is not inhibited (Di Bisceglie et al. 1995, Dusheiko et al. 1996, Felipe et al. 2000). This is in sharp contrast with the effect of ribavirin on hepatitis B in chronic HBV-infected patients where monotherapy with this drug results both in decreased ALT-levels as well as in HBV DNA-negativation although HBV viral rebound occurs in most cases after cessation of the treatment (Galban-Garcia et al. 2000, Fried et al. 1994). When combined with interferon alfa, ribavirin resulted in an increased frequency of sustained virological response (i.e., no HCV RNA detectable) after cessation of the combination therapy (Reichard et al. 1998, Lai et al. 1996). Reduction of the rate of viral rebound is believed to be the major contribution of ribavirin in combination therapies (Walker et al. 2003). Ribavirin therefore is not an antiviral agent and should be considered as an interferon-enhancer.

A side-effect of ribavirin treatment, i.e., ribavirin-related hemolysis or hemolytic anemia, may be ameliorated by combination of treatment with ribavirin with an antioxidant therapy (US 2003/00055013). Antioxidants as ameliorants of ribavirin-induced hemolysis or anemia include S-adenosyl-methionine, vitamin A, vitamin E, vitamin C, coenzyme-Q10, N-acetylcysteine, selenium, panavir, silybum marianum, lycopene, butylated hydroxyanisole, butylated hydroxytolune (e.g., WO 00/62799, US 2003/00055013). US 2003/0032590 discloses that erythropoetin decreased ribavirin-induced anemia and thus acts as an ameliorant of ribavirin-induced hemolysis or anemia. The L-isomer of ribavirin known as levovirin is a variant of ribavirin. Levovirin can not be metabolized to its triphosphate form which decreases the hemolytic activity of levovirin as compared to ribavirin. The ribavirin prodrug viramidine was also reported to have a decreased toxicity.

Ribavirin derivatives have been disclosed in, e.g., WO 01/81359 and US 6,277,830. ANA-246 (Anadys) is a ribavirin-like compound claimed to be less toxic than ribavirin.

Ribavirin which also acts as an inhibitor of inosine 5'-monophosphate dehydrogenase (IMPDH) may be replaced by another IMPDH inhibitor such as a pharmaceutically acceptable salt or prodrug of mycophenolic acid. An example of such a prodrug is mycophenolate mofetil (MFF), the morpholinoethyl ester of mycophenolic acid (US 2001/0046957; MFF is also known as CellCept, Roche Holdings). Other IMPDH inhibitors being tested for treatment of HCV infection include merimepodib or VX-497 (Vertex Pharmaceuticals, Sorbera et al. 2000), and a prodrug of ribavirin known as viramidine (Watson et al. 2002), and mizoribine monophosphate (Di Bisceglie et al. 2002). The L-isomer of ribavirin known as levovirin is not an inhibitor of IMPDH.

The most widely known and accepted HCV combination therapy today comprises combined administration of interferon (alfa or beta or a variant thereof) and ribavirin. Combination therapy of a CIFN with ribavirin is described by Pockros et al. (2003), da Silva et al. (2002) and Barbaro and Barbarini (2002).

Viral polymerase, such as HCV NS5B viral polymerase, inhibitor compounds have been disclosed in, e.g., WO 03/010141, WO 03/010140, WO 02/057425, and WO 02/032414. The HCV NS5B polymerase is also referred to as the replicase.

Inhibitors of viral proteases such as the HCV NS3-NS4A protease (required for proteolytic separation of the HCV NS3 and HCV NS4A proteins) have been disclosed in, e.g., WO 03/035060.

Inhibitors of viral proteases such as the HCV NS3 protease have been disclosed in, e.g., US 6,323,180, US 6,329,379, US 6,329,417, US 6,410,531, US 6,420,380, US 6,534,523, US 2002/0016442, US 2002/0037998, WO 99/07734, WO 00/09543 and WO 00/09558. More particularly, this series of patents and patent applications discloses tri-peptide inhibitors of HCV NS3 protease activity and a method of treating viral HCV infection using said tri-peptide inhibitors alone or in combination with other anti-HCV agents (alfa- or beta-interferon, ribavirin, amantadine) or inhibitors of other HCV targets (helicase, polymerase, metalloproteinase, IRES).

US 2002/0068702 and WO 02/08251 disclose HCV protease inhibitors of peptidic nature containing 11 amino acid residues. US 2002/0102235 discloses imidazolidinone compounds with HCV protease inhibitor activity. US 2002/0160962 discloses peptide inhibitors of HCV protease activity.

Inhibitors of the HCV IRES (internal ribosome entry site) include antisense oligonucleotides, (hammerhead) ribozymes, RNAi molecules, small hairpin RNA (shRNA) molecules and short interfering RNAs (siRNA). Such inhibitors may also be used to target HCV RNA sites other than the IRES. Any conserved region in the HCV genome is an attractive target for the development of such inhibitors.

Inhibitors of viral helicase, such as the HCV NS3 helicase, have been disclosed in, e.g., WO 01/07027.

The efficacy of a number of HCV IRES-, HCV NS3- and HCV NS5B-inhibitors is currently being evaluated in clinical studies in different phases (see Table 2 in Tan et al. 2002).

Other compounds may include peptides, antibodies or other molecules inhibiting the viral fusion or receptor domains, which are expected to be located on the envelope proteins. E.g. the interaction of the HCV envelope or either one or both of the envelope E1 and E2 proteins with L-SIGN, DC-SIGN (Pohlmann et al. 2003), apolipoprotein B, annexin V, tubulin (WO 99/24054), lactoferrin (WO 02/77239), a putative receptor called Eo (US 2002/0160936, WO 01/22984), a nuclear transport receptor of the Core protein (WO 02/22812), CD81 (WO 99/18198), the human scavenger receptor class B type I (Scarcelli et al. 2002), and cell surface heparan sulfate proteoglycans (Barth et al. 2002) are all potential targets.

Still other antiviral compounds may target protein interactions during conformational changes, viral assembly, or viral uncoating. Examples include without limitation the core-core, core-RNA, E1-E1, E1-E2, E1-Core, E2-Core and NS3-NS5A. interactions. It is suggested that NS4B may play an important role in viral assembly; therefore antivirals targetting NS4B may prevent maturation and/or viral budding. A method for analysis of interaction between HCV viral proteins is disclosed in US 2002/0102534. A method for identifying HCV entry factors is disclosed in EP 1 267 167, one possible entry factor being the LDL receptor. Compounds interfering with the association of HCV proteins with membranes are other candidates of antiviral agents (e.g., WO 02/089731).

Polyclonal or monoclonal antibodies containing anti-envelope antibodies could also reduce the viral titer and are to be regarded as antiviral agents herein.

### Results leading to the invention

The efficacy of combining an HCV vaccine with other non-vaccine HCV therapies was explored for the first time in chronic HCV-infected patients as described in the Examples of the present invention. The combination therapy comprised combination of an HCV vaccine with an antiviral agent. In particular, the combination therapy comprised combination of an HCV vaccine comprising an HCV E1 protein with an antiviral agent. In the current study, the antiviral agent consisted of interferon and the interferon-enhancer ribavirin. It is known that HCV E1 vaccination monotherapy only has no effect on HCV viral load (see, e.g., WO02/055548). It is further known that only a subset of chronic HCV-infected patients responds to interferon/ribavirin in the sense that HCV viral RNA is becoming undetectable. In view of this knowledge, a first unexpected finding of the present invention was that the number of chronic HCV patients displaying a virological response could be increased by combination of E1 vaccination with interferon/ribavirin (Examples 1 and 4). Likewise, the combination of E1 vaccination with interferon/ribavirin had a surprising beneficial effect on, i.e. repressed, HCV viral breakthrough during therapy (Example 2). A similar beneficial effect was observed at the level of HCV rebound after cessation of the E1 vaccination combined with interferon/ribavirin (Example 3). The observed beneficial effects of E1 vaccination on top of interferon/ribavirin therapy could be related to immune responses (Example 5). Furthermore, it could be demonstrated that the combination of interferon/ribavirin does not enhance E1-induced immune responses to HCV (Example 6). The importance of anti-HCV immune responses in clearance of HCV from chronically infected patients is further demonstrated in Example 7. The inventors reasoned that the E1 immune responses seen during interferon-ribavirin therapy, might be related to the immune responses of subjects who previously cleared HCV during IFN-based therapy. It could indeed be demonstrated that similar E1 immune responses could be recalled by injecting a small dose of E1 protein in 3 persons with a cleared infection. Therefore it is concluded that E1 immune responses contribute to clearance of HCV before and/or during and/or after antiviral therapy.

Further advantages of any new combination therapy generally rely on the possibility to decrease the amounts of the different components of the combination therapy and, thus, the possibility to reduce the side-effects linked to the different components. Another aim of improving combination therapies is to reduce treatment time and thus alleviate the burden for the patients due to side effects. Another advantage of new combination therapies lie in the combination of compounds combating HCV via different modes of action hence decreasing the chances of HCV being able to overcome the action of any individual compound. Indeed it is anticipated that viral resistance against the new antiviral compounds in development, will emerge rapidly. Based on the high mutation rate of HCV, it is anticipated that viral resistance will appear between 1 and 36 months of antiviral treatment, more preferably between 2 and 12 months of antiviral therapy.

Following from the foregoing, a first aspect of the invention relates to the use of an isolated HCV E1 protein and/or of an HCV E1 DNA vector for the manufacture of an HCV vaccine or an HCV immunogenic composition
- for therapeutic treatment of an HCV-infected mammal; and/or
- for inducing an immune response in an HCV-infected mammal; and/or
- for enhancing HCV viral clearance in an HCV-infected mammal; and/or
- for suppressing HCV viral breakthrough in an HCV-infected mammal; and/or
- for suppressing HCV viral rebound after cessation of therapeutic treatment of an HCV-infected mammal;
by a method comprising administering said HCV vaccine before and/or during and/or after a therapy comprising or with at least one antiviral agent.

Alternatively, the use of at least one antiviral agent for the manufacture of a pharmaceutical composition
- for therapeutic treatment of an HCV-infected mammal; and/or
- for inducing an immune response in an HCV-infected mammal; and/or
- for enhancing HCV viral clearance in an HCV-infected mammal; and/or
- for suppressing HCV viral breakthrough in an HCV-infected mammal; and/or
- for suppressing HCV viral rebound after cessation of therapeutic treatment of an HCV-infected mammal
by a method comprising administering said pharmaceutical composition before and/or during and/or after a therapy comprising administering an HCV vaccine or an HCV immunogenic composition wherein said HCV vaccine or HCV immunogenic composition is at least comprising an isolated HCV E1 protein and/or of an HCV E1 DNA vector as active substance.

A further alternative aspect of the invention relates to the use of an isolated HCV E1 protein and/or of an HCV E1 DNA vector for the manufacture of an HCV vaccine or an HCV immunogenic composition for therapeutic treatment of an HCV-infected mammal by a method comprising administering said HCV vaccine before and/or during and/or after a therapy comprising or with at least one antiviral agent wherein said therapeutic treatment (i) induces an immune response in said HCV-infected mammal and/or (ii) enhances HCV viral clearance in said HCV-infected mammal and/or (iii) suppresses HCV viral breakthrough in said HCV-infected mammal; and/or (iv) wherin HCV viral rebound is suppressed after cessation of said therapeutic treatment.

A yet another alternative aspect the invention relates to the use of at least one antiviral agent for the manufacture of a pharmaceutical composition for therapeutic treatment of an HCV-infected mammal by a method comprising administering said pharmaceutical composition before and/or during and/or after a therapy comprising administering an HCV vaccine or HCV immunogenic composition wherein said HCV vaccine or said HCV immunogenic composition is at least comprising an isolated HCV E1 protein and/or of an HCV E1 DNA vector as active substance; and wherein said therapeutic treatment (i) induces an immune response in said HCV-infected mammal and/or (ii) enhances HCV viral clearance in said HCV-infected mammal and/or (iii) suppresses HCV viral breakthrough in said HCV-infected mammal; and/or (iv) wherin HCV viral rebound is suppressed after cessation of said therapeutic treatment.

The immune response is an immune response to HCV and is a humoral and/or cellular immune response. The immune response is at least to an HCV E1 protein. Mammal include non-human primates and humans.

In specific embodiments to any of the recited uses said at least one antiviral agent may be an interferon or a variant or consensus sequence thereof, or an inducer of interferon. Said interferon may be a type I or a type III interferon or a variant or consensus sequence of any thereof. In particular, said type I interferon is chosen from an interferon-alfa, interferon-beta, interferon-omega or interferon-tau or said type III interferon is chosen from IL-28A, IL-28B or IL-29. Said inducer of interferon may be chosen from TLR7-ligands, TLR-9 ligands, ANA245, or ANA971.

Another embodiment relates to the at least one antiviral agent that may be chosen from an IMPDH-inhibitor, an anti-HCV antibody, an immune modulator, a HCV-receptor inhibitor, an HCV fusion inhibitor, a modulator of host cell function required for HCV replication, a molecule targeting a step in the HCV life cycle, a molecule targeting an HCV IRES, a molecule binding to HCV RNA, a molecule targeting an HCV NS2-NS3 protease, a molecule targeting an HCV NS3 protease, a molecule targeting an HCV NS3 RNA helicase, a molecule targeting an HCV NS3-NS4A protease, a molecule targeting an HCV NS5B RNA-dependent RNA polymerase, and a molecule inhibiting binding of HCV E2 or HCV NS5 to protein kinase R, a molecule targeting an HCV Core protein, a molecule targeting an HCV E1 protein or a molecule targeting an HCV E2 protein

In a further aspect of the invention and if said at least one antiviral agent in the uses recited in the first aspect of the invention is interferon or a variant or consensus sequence thereof, the methods of said uses may further comprise a therapy comprising or with an interferon-enhancer or at least one additional antiviral agent.

In a specific embodiment thereto, said interferon-enhancer is chosen from interferon gamma, ribavirin, levovirin, viramidine, amantadine, thymosin alfa-1, histamine dihydrochloride, a methyldonor, or ANA246.

In another specific embodiment, said at least one additional antiviral agent is chosen from an IMPDH-inhibitor, an anti-HCV antibody, an immune modulator, a HCV-receptor inhibitor, an HCV fusion inhibitor, a modulator of host cell function required for HCV replication, a molecule targeting a step in the HCV life cycle, a molecule targeting an HCV IRES, a molecule binding to HCV RNA, a molecule targeting an HCV NS2-NS3 protease, a molecule targeting an HCV NS3 protease, a molecule targeting an HCV NS3 RNA helicase, a molecule targeting an HCV NS3-NS4A protease, a molecule targeting an HCV NS5B RNA-dependent RNA polymerase, and a molecule inhibiting binding of HCV E2 or HCV NS5 to protein kinase R, a molecule targeting an HCV Core protein, a molecule targeting an HCV E1 protein or a molecule targeting an HCV E2 protein.

Said IMPDH-inhibitor may be chosen from ribavirin or a variant thereof, viramidine, mizoribine monophosphate, merimepodib or mycophenolic acid or a variant thereof.

If the interferon-enhancer or IMPDH-inhibitor applied in any of the above uses is ribavirin or a variant thereof, then administration of an ameliorant of ribavirin-induced anemia to the HCV-infected mammal is a further aspect of the invention. Said ameliorant of ribavirin-induced anemia may be an antioxidant or erythropoietin.

Another aspect of the invention covers a pharmaceutical pack or kit at least comprising one or more containers with an HCV vaccine and one or more containers with at least one antiviral agent and wherein said HCV vaccine is at least comprising an isolated HCV E1 protein and/or an HCV E1 DNA vector as active substance.

In one embodiment, said at least one antiviral agent is an interferon or a variant or consensus sequence thereof or an inducer of interferon.

In a specific embodiment to the uses of the invention and the pharmaceutical packs or kits of the invention, the HCV E1 protein may be an E1s protein or the HCV E1 DNA vector may be encoding an E1s protein. Said HCV E1 protein may be the HCV E1 protein defined by SEQ ID NO:1 or said HCV E1 DNA vector may be encoding the HCV E1 protein defined by SEQ ID NO:1.

In another specific embodiment to the uses of the invention and the pharmaceutical packs or kits of the invention said HCV E1 protein may be added to said HCV vaccine as a viral-like particle.

In a further specific embodiment to the uses of the invention and the pharmaceutical packs or kits of the invention the cysteine-thiol groups in said HCV E1 protein may be reversibly or irreversibly blocked.

The terminology in the aspects and embodiments of the invention as summarized above are explained further hereafter.

An immunogenic composition is a composition comprising an antigen (and/or a DNA vaccine vector encoding an antigen) capable of eliciting at least one element of the immune response against the antigen comprised in said composition when said composition is introduced into the body of an animal capable of raising an immune response. An immunogenic composition may comprise more than one antigen, i.e., a plurality of antigens, e.g. 2, 3, 4, 5, 6, 7, 8, 9, 10 or more, e.g., up to 15, 20, 25, 30, 40 or 50 or more distinct antigens. In particular, the immunogenic composition of the invention is an HCV immunogenic composition is comprising at least an isolated HCV E1 protein and/or at least an HCV E1 DNA vaccine vector as active substance. An immunogenic composition may additionally comprise one or more further active substances and/or at least one of a pharmaceutically acceptable carrier, adjuvant or vehicle.

A vaccine composition is an immunogenic composition capable of eliciting an immune response sufficiently broad and vigorous to provoke one or both of:
- a stabilizing effect on the multiplication of a pathogen already present in a host and against which the vaccine composition is targeted; and
- an increase of the rate at which a pathogen newly introduced in a host, after immunization with a vaccine composition targeted against said pathogen, is resolved from said host.
A vaccine composition may also provoke an immune response broad and strong enough to exert a negative effect on the survival of a pathogen already present in a host or broad and strong enough to prevent an immunized host from developing disease symptoms caused by a newly introduced pathogen. A vaccine composition may also induce an immune response in a host already infected with the pathogen against which the immune response leading to a halting or reversion of disease progression in the absence of eradication of the pathogen. In particular the vaccine composition of the invention is a HCV vaccine composition. The HCV vaccine composition is comprising at least an isolated HCV E1 protein and/or an HCV E1 DNA vaccine vector as active substance, in particular comprising an effective amount of said HCV E1 protein and/or of said HCV E1 DNA vaccine vector. An HCV vaccine composition may additionally comprise one or more further active substances and/or at least one of a pharmaceutically acceptable carrier, adjuvant or vehicle.

An effective amount of an antigen in a vaccine composition is referred to as an amount of antigen required and sufficient to elicit an immune response. The effective amount of a DNA vaccine vector is the amount of said vector required to reach an amount of produced antigen required and sufficient to elicit an immune response. It will be clear to the skilled artisan that the immune response sufficiently broad and vigorous to provoke the effects envisaged by the vaccine composition may require successive (in time) immunizations with the vaccine composition as part of a vaccination scheme or vaccination schedule. The "effective amount" may vary depending on the health and physical condition of the individual to be treated, the age of the individual to be treated (e.g. dosing for infants may be lower than for adults) the taxonomic group of the individual to be treated (e.g. human, non-human primate, primate, etc.), the capacity of the individual's immune system to mount an effective immune response, the degree of protection desired, the formulation of the vaccine, the treating doctor's assessment, the strain of the infecting pathogen and other relevant factors. It is expected that the effective antigen amount will fall in a relatively broad range that can be determined through routine trials. Usually, the antigen amount will vary from 0.01 to 1000 µg/dose, more particularly from 0.1 to 100 µg/dose. Dosage treatment may be a single dose schedule or a multiple dose schedule. The vaccine may be administered in conjunction with other immunoregulatory agents.

With HCV E1 protein is meant herein any protein comprising at least one epitope of the full-length HCV E1 protein. The term "HCV E1 protein" comprises the full-length E1 protein, fragments thereof and derivatives of any thereof. In particular, when comprised in an immunogenic composition or a vaccine composition, a HCV E1 protein is capable of eliciting an immune response as defined for an immunogenic composition or a vaccine composition respectively. The full-length HCV envelope E1 protein corresponds to the HCV polyprotein domain spanning amino acids 192-383. The HCV envelope E1s protein corresponds to the HCV polyprotein domain spanning amino acids 192-326; the E1s protein is an exemplary part of the E1 protein. An exemplary HCV type 1b E1 protein sequence is given in Figure 1 (SEQ ID NO:2) wherein the corresponding E1s part is also indicated (SEQ ID NO:1). A derivative of a HCV protein is meant to include HCV proteins comprising modified amino acids (e.g., conjugated with biotin or digoxigenin, non-natural amino acids), HCV proteins comprising insertions (relative to a naturally occurring HCV sequence) of one or more amino acid, HCV proteins wherein one or more amino acids have been deleted (relative to a naturally occurring HCV sequence), as well as fusion proteins. Fusion proteins may be formed between an HCV E1 protein and another HCV protein or between a HCV E1 protein and a non-HCV peptide or protein such as a B-cell epitope, a T-cell epitope, a CTL epitope or a cytokine. Other peptide or protein fusion partners include bovine serum album, keyhole limpet hemocyanin, soybean or horseradish peroxidase, beta-galactosidase, luciferase, alkaline phosphatase, glutathione S-transferase or dihydrofolate reductase or heterologous epitopes such as (histidine)₆-tag, protein A, maltose-binding protein, Tag• 100 epitope, c-myc epitope, FLAG®-epitope, lacZ, CMP (calmodulin-binding peptide), HA epitope, protein C epitope or VSV epitope.
The HCV immunogenic composition or HCV vaccine composition as used herein may comprise as active substances a plurality of HCV E1 proteins derived from different HCV genotypes, subtypes or isolates and, optionally at least one of a pharmaceutically acceptable carrier, adjuvant or vehicle. Alternatively, the E1 proteins may be derived from a consensus sequence for a given isolate. A consensus sequence for a given isolate is constructed as a consensus sequence of individual clones derived from a single serum sample from one chronic carrier. An isolate consensus sequence thus takes into account the quasispecies variability within an isolate. The HCV immunogenic composition or HCV vaccine composition as used herein may comprise a plurality of HCV E1 DNA vaccine vectors as active substances, or alternatively comprise as active substance at least one HCV E1 DNA vector comprising a plurality of HCV E1 protein open reading frames. Herein the plurality of resulting HCV E1 proteins (from a plurality of HCV E1 DNA vaccine vectors or from the at least one HCV E1 DNA vaccine vector) may be derived from different HCV genotypes, subtypes or isolates, or from different consensus sequences for a set of different isolates. A combination of multiple HCV E1 proteins as described above and of the at least one HCV E1 DNA vaccine vector or multiple HCV E1 DNA vaccine vectors as described above is another possible HCV immunogenic composition or HCV vaccine composition. Any of said immunogenic or vaccine compositions may additionally comprise one or more further active substances and/or at least one of a pharmaceutically acceptable carrier, adjuvant or vehicle.

Currently known HCV types include HCV genotypes 1, 2, 3, 4, 5, 6, 7, 8, 9, 10, 11 and known subtypes thereof include HCV subtypes 1a, 1b, 1c, 1d, 1e, 1f, 1g, 2a, 2b, 2c, 2d, 2e, 2f, 2g, 2h, 2i, 2k, 2l, 3a, 3b, 3c, 3d, 3e, 3f, 3g, 4a, 4b, 4c, 4d, 4e, 4f, 4g, 4h, 4i, 4j, 4k, 4l, 4m, 5a, 6a, 6b, 7a, 7b, 7c, 7d, 8a, 8b, 8c, 8d, 9a, 9b, 9c, 10a and 11a. The sequences of cDNA clones covering the complete genome of several prototype isolates have been determined and include complete prototype genomes of the HCV genotypes 1a (e.g., GenBank accession number AF009606), 1b (e.g., GenBank accession number AB016785), 1c (e.g., GenBank accession number D14853), 2a (e.g., GenBank accession number AB047639), 2b (e.g., GenBank accession number AB030907), 2c (e.g., GenBank accession number D50409) 2k (e.g., GenBank accession number AB031663), 3a (e.g., GenBank accession number AF046866), 3b (e.g., GenBank accession number D49374), 4a (e.g., GenBank accession number Y11604), 5a (e.g., GenBank accession number AF064490), 6a (e.g., GenBank accession number Y12083), 6b (e.g., GenBank accession number D84262), 7b (e.g., GenBank accession number D84263), 8b (e.g., GenBank accession number D84264), 9a (e.g., GenBank accession number D84265), 10a (e.g., GenBank accession number D63821) and 11a (e.g., GenBank accession number D63822). A new HCV genotype was further described in International Patent Publication No. WO03/20970. An HCV isolate is to be considered as a HCV quasispecies isolated from a HCV-infected mammal. A HCV quasispecies usually comprises a number of variant viruses with variant genomes usually of the same HCV type or HCV subtype.

The immunogenic composition or vaccine composition as used in the current invention may comprise DNA vaccine vectors capable of expressing or effectuating expression of an antigen, or may comprise such DNA vaccine vectors in addition to the antigen. Particularly relating to the current invention, the HCV immunogenic composition or HCV vaccine composition may comprise DNA vaccine vectors capable of expressing or effectuating expression of at least one or more HCV E1 proteins; or the HCV immunogenic composition or HCV vaccine composition may comprise an isolated HCV E1 protein in combination with an HCV E1 DNA vaccine vector. Alternatively, the protein- or peptide-based immunogenic composition or vaccine composition of the invention may be used in combination with a DNA vector-based immunogenic composition or vaccine composition (also referred to as "DNA vaccine" or "HCV DNA vaccine" if the DNA vector comprised therein is encoding a HCV protein or part thereof). Such combination for instance includes a DNA-prime protein-boost vaccination scheme wherein vaccination is initiated by administering a DNA vector-based immunogenic composition or vaccine composition and is followed by administering a protein- or peptide-based immunogenic composition or vaccine composition of the invention. In particular the DNA vaccine vector is capable of expressing at least one or more HCV E1 proteins.

With a "DNA vector" or "DNA vaccine vector" is meant any DNA molecule carrying or comprising at least the open reading frame for one or more E1 proteins as defined above. In general, said open reading frames are operably linked to the same or different transcription regulatory elements, such as promoters and terminators. The transcription regulatory elements enable expression (in a vertebrate or vertebrate cell) of the peptide(s) encoded by the open reading frame. Multiple proteins may be translated from a single transcript by means of internal ribosome entry sites (IRESs) in the transcript. The terms "DNA vector" or "DNA vaccine vector" are meant to include naked linear DNA, naked plasmid DNA, or linear or plasmid DNA formulated with at least one of a suitable pharmaceutically acceptable carrier, adjuvant or vehicle. DNA vectors or DNA vaccine vectors further comprise recombinant viruses (e.g., vaccinia-, alpha- or adenoviruses, canary pox virus, Semliki Forest virus, avipox virus, Ankara Modified Virus) or bacteria (e.g., (attenuated) *Salmonella typhimurium* as used in oral DNA vaccination) or recombinant viruses or bacteria formulated with at least one of a suitable pharmaceutically acceptable carrier, adjuvant or vehicle. An overview of DNA vaccines and their administration route may be found in Alpar and Bramwell (2002). A "HCV E1 DNA vector" or "HCV E1 DNA vaccine vector" relates to any DNA carrier comprising at least the open reading frame(s) for one or more HCV E1 proteins.

As used herein, the term "transcription regulatory elements" refers to a nucleotide sequence which contains essential regulatory elements, such that upon introduction into a living vertebrate cell it is able to direct the cellular machinery to produce transcription products encoded by the polynucleotide.

The term "operably linked" refers to a juxtaposition wherein the components are configured so as to perform their usual function. Thus, transcription regulatory elements operably linked to a nucleotide sequence are capable of effecting the expression of said nucleotide sequence. Those skilled in the art can appreciate that different transcriptional promoters, terminators, carrier vectors or specific gene sequences may be used successfully

A "pharmaceutically acceptable carrier" or "pharmaceutically acceptable adjuvant" is any suitable excipient, diluent, carrier and/or adjuvant which, by themselves, do not induce the production of antibodies harmful to the individual receiving the composition nor do they elicit protection. Preferably, a pharmaceutically acceptable carrier or adjuvant enhances the immune response elicited by an antigen. Suitable carriers or adjuvantia typically comprise one or more of the compounds included in the following non-exhaustive list:
- large slowly metabolized macromolecules such as proteins, polysaccharides, polylactic acids, polyglycolic acids, polymeric amino acids, amino acid copolymers and inactive virus particles;
- aluminium hydroxide, aluminium phosphate (see International Patent Application Publication No. WO93/24148), alum (KAl(SO₄)₂.12H₂O), or one of these in combination with 3-0-deacylated monophosphoryl lipid A (see International Patent Application Publication No. WO93/19780);
- N-acetyl-muramyl-L-threonyl-D-isoglutamine (see U.S. Patent No. 4,606,918), N-acetyl-normuramyl-L-alanyl-D-isoglutamine, N-acetylmuramyl-L-alanyl-D-isoglutamyl-L-alanine2-(1',2'-dipalmitoyl-sn-glycero-3 -hydroxyphosphoryloxy) ethylamine;
- RIBI (ImmunoChem Research Inc., Hamilton, MT, USA) which contains monophosphoryl lipid A (i.e., a detoxified endotoxin), trehalose-6,6-dimycolate, and cell wall skeleton (MPL + TDM + CWS) in a 2% squalene/Tween 80 emulsion. Any of the three components MPL, TDM or CWS may also be used alone or combined 2 by 2.; (moved to end to cover more combinations)
- adjuvants such as Stimulon (Cambridge Bioscience, Worcester, MA, USA), SAF-1 (Syntex);
- adjuvants such as combinations between QS21 and 3-de-O-acetylated monophosphoryl lipid A (see International Patent Application Publication No. WO94/00153) which may be further supplemented with an oil-in-water emulsion (see, e.g., International Patent Application Publication Nos. WO95/17210, WO97/01640 and WO9856414) in which the oil-in-water emulsion comprises a metabolisable oil and a saponin, or a metabolisable oil, a saponin, and a sterol, or which may be further supplemented with a cytokine (see International Patent Application Publication No. WO98/57659);
- adjuvants such as MF-59 (Chiron), or poly[di(carboxylatophenoxy) phosphazene] based adjuvants (Virus Research Institute);
- blockcopolymer based adjuvants such as Optivax (Vaxcel, Cytrx) or inulin-based adjuvants, such as Algammulin and Gammalnulin (Anutech);
- Complete or Incomplete Freund's Adjuvant (CFA or IFA, respectively) or Gerbu preparations (Gerbu Biotechnik). It is to be understood that Complete Freund's Adjuvant (CFA) may be used for non-human applications and research purposes as well;
- a saponin such as QuilA, a purified saponin such as QS21, QS7 or QS17, β-escin or digitonin;
- immunostimulatory oligonucleotides comprising unmethylated CpG dinucleotides such as [purine-purine-CG-pyrimidine-pyrimidine] oligonucleotides. These immunostimulatory oligonucleotides include CpG class A, B, and C molecules (Coley Pharmaceuticals), ISS (Dynavax), Immunomers (Hybridon). Immunostimulatory oligonucleotides may also be combined with cationic peptides as described, e.g., by Riedl et al. (2002);
- Immune Stimulating Complexes comprising saponins, for example Quil A (ISCOMS);
- excipients and diluents, which are inherently non-toxic and non-therapeutic, such as water, saline, glycerol, ethanol, wetting or emulsifying agents, pH buffering substances, preservatives, and the like;
- a biodegradable and/or biocompatible oil such as squalane, squalene, eicosane, tetratetracontane, glycerol, peanut oil, vegetable oil, in a concentration of, e.g., 1 to 10% or 2.5 to 5%;
- vitamins such as vitamin C (ascorbic acid or its salts or esters), vitamin E (tocopherol), or vitamin A;
- carotenoids, or natural or synthetic flavanoids;
- trace elements, such as selenium;
- any Toll-like receptor ligand as reviewed in Barton and Medzhitov (2002).
Any of the afore-mentioned adjuvants comprising 3-de-O-acetylated monophosphoryl lipid A, said 3-de-O-acetylated monophosphoryl lipid A may be forming a small particle (see International Patent Application Publication No. WO94/21292).
In any of the aforementioned adjuvants MPL or 3-de-O-acetylated monophosphoryl lipid A can be replaced by a synthetic analogue referred to as RC-529 or by any other amino-alkyl glucosaminide 4-phosphate (Johnson et al. 1999, Persing et al. 2002). Alternatively it can be replaced by other lipid A analogues such as OM-197 (Byl et al., 2003);

A "pharmaceutically acceptable vehicle" includes vehicles such as water, saline, physiological salt solutions, glycerol, ethanol, etc. Auxiliary substances such as wetting or emulsifying agents, pH buffering substances, preservatives may be included in such vehicles.

Typically, a vaccine composition is prepared as an injectable, either as a liquid solution or suspension. Injection may be subcutaneous, intramuscular, intravenous, intraperitoneal, intrathecal, intradermal, intraepidermal. Other types of administration comprise implantation, suppositories, oral ingestion, enteric application, inhalation, aerosolization or nasal spray or drops. Solid forms, suitable for dissolving in, or suspension in, liquid vehicles prior to injection may also be prepared. The preparation may also be emulsified or encapsulated in liposomes for enhancing adjuvant effect.

A HCV E1 protein may be of synthetic origin, i.e. synthesized by applying organic chemistry, or of recombinant origin. HCV E1 protein may be produced by expression in, e.g., mammalian or insect cells infected with recombinant viruses, yeast cells or bacterial cells.

More particularly, said mammalian cells include HeLa cells, Vero cells, RK13 cells, MRC-5 cells, Chinese hamster ovary (CHO) cells, Baby hamster kidney (BHK) cells and PK15 cells.

More particularly, said insect cells include cells of *Spodoptera frugiperda,* such as Sf9 cells.

More particularly, said recombinant viruses include recombinant vaccinia viruses, recombinant adenoviruses, recombinant baculoviruses, recombinant canary pox viruses, recombinant Semlike Forest viruses, recombinant alphaviruses, recombinant Ankara Modified viruses and recombinant avipox viruses.

More particularly, said yeast cells include cells of *Saccharomyces,* such as *Saccharomyces cerevisiae, Saccharomyces kluyveri,* or *Saccharomyces uvarum, Schizosaccharomyces,* such as *Schizosaccharomyces pombe, Kluyveromyces,* such as *Kluyveromyces lactis, Yarrowia,* such as *Yarrowia lipolytica, Hansenula,* such as *Hansenula polymorpha, Pichia,* such as *Pichia pastoris, Aspergillus species, Neurospora,* such as *Neurospora crassa,* or *Schwanniomyces,* such as *Schwanniomyces occidentalis,* or mutant cells derived from any thereof. More specifically, the HCV peptide or part thereof according to the invention is the product of expression in a *Hansenula* cell.

More particularly, said bacterial cells include cells of *Escherichia coli* or *Streptomyces* species.

In the HCV E1 protein as described herein, one cysteine residue, or 2 or more cysteine residues comprised in said peptides may be "reversibly or irreversibly blocked".

An "irreversibly blocked cysteine" is a cysteine of which the cysteine thiol-group is irreversibly protected by chemical means. In particular, "irreversible protection" or "irreversible blocking" by chemical means refers to alkylation, preferably alkylation of a cysteine in a protein by means of alkylating agents, such as, for example, active halogens, ethylenimine or N-(iodoethyl)trifluoro-acetamide. In this respect, it is to be understood that alkylation of cysteine thiol-groups refers to the replacement of the thiol-hydrogen by (CH₂)ₙR, in which n is 0, 1, 2, 3 or 4 and R= H, COOH, NH₂, CONH₂ , phenyl, or any derivative thereof. Alkylation can be performed by any method known in the art, such as, for example, active halogens X(CH₂)ₙR in which X is a halogen such as I, Br, Cl or F. Examples of active halogens are methyliodide, iodoacetic acid, iodoacetamide, and 2-bromoethylamine.

A "reversibly blocked cysteine" is a cysteine of which the cysteine thiol-groups is reversibly protected. In particular, the term "reversible protection" or "reversible blocking" as used herein contemplates covalently binding of modification agents to the cysteine thiol-groups, as well as manipulating the environment of the protein such, that the redox state of the cysteine thiol-groups remains (shielding). Reversible protection of the cysteine thiol-groups can be carried out chemically or enzymatically.

The term "reversible protection by enzymatical means" as used herein contemplates reversible protection mediated by enzymes, such as for example acyl-transferases, e.g. acyl-transferases that are involved in catalysing thio-esterification, such as palmitoyl acyltransferase.

The term "reversible protection by chemical means" as used herein contemplates reversible protection:
1. by modification agents that reversibly modify cysteinyls such as for example by sulphonation and thio-esterification;
2. by modification agents that reversibly modify the cysteinyls of the present invention such as, for example, by heavy metals, in particular Zn²⁺, Cd²⁺, mono-, dithio- and disulfide-compounds (e.g. aryl- and alkylmethanethiosulfonate, dithiopyridine, dithiomorpholine, dihydrolipoamide, Ellmann reagent, aldrothiol™ (Aldrich) (Rein et al. 1996), dithiocarbamates), or thiolation agents (e.g. gluthathion, N-Acetyl cysteine, cysteineamine). Dithiocarbamate comprise a broad class of molecules possessing an R₁R₂NC(S)SR₃ functional group, which gives them the ability to react with sulphydryl groups. Thiol containing compounds are preferentially used in a concentration of 0.1-50 mM, more preferentially in a concentration of 1-50 mM, and even more preferentially in a concentration of 10-50 mM;
3. by the presence of modification agents that preserve the thiol status (stabilise), in particular antioxidantia, such as for example DTT, dihydroascorbate, vitamins and derivates, mannitol, amino acids, peptides and derivates (e.g. histidine, ergothioneine, carnosine, methionine), gallates, hydroxyanisole, hydoxytoluene, hydroquinon, hydroxymethylphenol and their derivates in concentration range of 10 µM-10 mM, more preferentially in a concentration of 1-10 mM;
4. by thiol stabilising conditions such as, for example, (i) cofactors as metal ions (Zn²⁺, Mg²⁺), ATP, (ii) pH control (e.g. for proteins in most cases pH ∼5 or pH is preferentially thiol pKₐ -2; e.g. for peptides purified by Reversed Phase Chromatography at pH ∼2).
Combinations of reversible protection as described in (1), (2), (3) and (4) may be applied.

The reversible protection and thiol stabilizing compounds may be presented under a monomeric, polymeric or liposomic form.

The removal of the reversibly protection state of the cysteine residues can chemically or enzymatically accomplished by e.g.:
- a reductant, in particular DTT, DTE, 2-mercaptoethanol, dithionite, SnCl₂, sodium borohydride, hydroxylamine, TCEP, in particular in a concentration of 1-200 mM, more preferentially in a concentration of 50-200 mM;
- removal of the thiol stabilising conditions or agents by e.g. pH increase;
- enzymes, in particular thioesterases, glutaredoxine, thioredoxine, in particular in a concentration of 0.01-5 µM, even more particular in a concentration range of 0.1-5 µM.;
- combinations of the above described chemical and/or enzymatical conditions.

The removal of the reversibly protection state of the cysteine residues can be carried out *in vitro* or *in vivo,* e.g. in a cell or in an individual.

The terms "oligomeric particle", "virus-like particle", "viral-like particle", or "VLP" is herein defined as structures of a specific nature and shape containing several HCV E1 envelope proteins. It should be clear that the particles of the present invention are defined to be devoid of infectious HCV RNA genomes. The particles of the present invention can be higher-order particles of spherical nature which can be empty, consisting of a shell of envelope proteins in which lipids, detergents, the HCV core protein, or adjuvant molecules can be incorporated. The latter particles can also be encapsulated by liposomes or apolipoproteins, such as, for example, apolipoprotein B or low density lipoproteins, or by any other means of targeting said particles to a specific organ or tissue. In this case, such empty spherical particles are often referred to as "virus-like particles" or VLPs. Alternatively, the higher-order particles can be solid spherical structures, in which the complete sphere consists of HCV envelope protein oligomers, in which lipids, detergents, the HCV core protein, or adjuvant molecules can be additionally incorporated, or which in turn may be themselves encapsulated by liposomes or apolipoproteins, such as, for example, apolipoprotein B, low density lipoproteins, or by any other means of targeting said particles to a specific organ or tissue, e.g. asialoglycoproteins. The particles can also consist of smaller structures (compared to the empty or solid spherical structures indicated above) which are usually round (see further)-shaped and which usually do not contain more than a single layer of HCV envelope proteins. A typical example of such smaller particles are rosette-like structures which consist of a lower number of HCV envelope proteins, usually between 4 and 16. A specific example of the latter includes the smaller particles obtained with E1s in 0.2% CHAPS as exemplified herein which apparently contain 8-10 monomers of E1s. Such rosette-like structures are usually organized in a plane and are round-shaped, e.g. in the form of a wheel. Again lipids, detergents, the HCV core protein, or adjuvant molecules can be additionally incorporated, or the smaller particles may be encapsulated by liposomes or apolipoproteins, such as, for example, apolipoprotein B or low density lipoproteins, or by any other means of targeting said particles to a specific organ or tissue. Smaller particles may also form small spherical or globular structures consisting of a similar smaller number of HCV envelope proteins in which lipids, detergents, the HCV core protein, or adjuvant molecules could be additionally incorporated, or which in turn may be encapsulated by liposomes or apolipoproteins, such as, for example, apolipoprotein B or low density lipoproteins, or by any other means of targeting said particles to a specific organ or tissue. The size (i.e. the diameter) of the above-defined particles, as measured by the well-known-in-the-art dynamic light scattering techniques, is usually between 1 to 100 nm, more preferentially between 2 to 70 nm. Virus-like particles of HCV envelope proteins have been described in International Patent Application Publication Nos. WO99/67285, WO02/055548 and in International Patent Publication No. WO02/086101.

A pharmaceutical pack or kit is a pack or kit containing one or more pharmaceutical compositions, pharmaceutical agents or pharmaceutical compounds. Pharmaceutical compositions, pharmaceutical agents or pharmaceutical compounds include vaccine compositions and compositions comprising an antiviral agent independent of their formulation (e.g., solution, tablet, powder, capsule etc.) and their packaging (e.g., vial, aseptically sealed vial, bottle, screw-capped bottle, syringe, tablet or capsule strips etc.). The package wherein a pharmaceutical composition, agent or compound is present is herein referred to the general term "container". A pharmaceutical pack or kit may comprise further elements such as one or more empty syringes, a pack or kit insert indicating (e.g., listing indications and contra-indications) and/or a chart for marking adherence to a therapy comprising administration of the pharmaceutical compositions, agents or compounds present in the pharmaceutical pack or kit. Pharmaceutical compositions or components of a pharmaceutical pack or kit may be administered by the same or different routes and include oral, rectal, intravenous, intramuscular, subcutaneous, intra-articular, inhalation, topical or transdermal, vaginal, and ophthalmic administration.

### EXAMPLES

### EXAMPLE 1. Use of E1 vaccination to induce late biochemical or viral response during IFN-treatment.

In a phase I/II, prospective, open label, single center, exploratory study, 7 patients who remained HCV-RNA positive despite at least 10 weeks of IFN treatment combined with ribavirin (see Table 1 for baseline characteristics and description of interferon/ribavirin therapy) were given a course of E1 vaccinations. This course consisted of 6 intra-muscular immunizations with 20 µg E1 adsorbed on 0.13% of alum (the E1 protein was expressed in and purified from mammalian cells and further converted into oligomeric particles or viral-like particles as described in Example 19 of PCT/EP02/14480). This therapeutic vaccine was administered with a 3-week interval, while the interferon-ribavirin treatment was continued. Both the viral and biochemical responses were analyzed before, during and after the E1 immunization. Viral response was measured with quantitative (Amplicor HCV Monitor 2.0, Roche, BranchBurg, NJ, USA), and/or qualitative methods (Amplicor HCV Amplification kit 2.0/Cobas Amplicon Detection kit 2.0, Roche). Biochemical responses were assessed by measuring ALT using standard methods.

Remarkably, 2 out of 7 patients resolved their infection while receiving E1 vaccinations, patient 01002 at week 27, and patient 01010 at week 16 (Table 1, weeks recalculated from start of IFN-based therapy). Although the number of patients in this study is small this frequency of late RNA resolution, both by quantitative as well as qualitative methods, is high compared to what is known from other studies without additional vaccination, especially the case resolving HCV at week 27 is an unexpected fmding since in several studies no increased numbers of loss of HCV-RNA were noted comparing groups receiving 24 or 48 weeks of interferon-ribavirin treatment (Poynard et al. 1998, Table 2 therein; McHutchison et al. 1998, Table 2 therein). On the contrary loss of HCV-RNA observed at week 48 seems to be somewhat lower which may be a consequence of breakthrough infections occurring between week 24 and week 48. The studies of Poynard and McHutchison do not give data of frequency of loss of HCV-RNA at week 12. The study of Pol and colleagues (2000, Fig 4 therein) in genotype 1b patients does however give these data and reveals that almost no overall increase in viral clearing is observed between week 12 and week 48 of treatment with interferon/ribavirin (comparing a frequency of 58% in group C4 at M3 (which equaling week 12) with a frequency of 59% in group C12 at M12 (which equaling week 48)). In addition also in this study, the number of patients showing viral RNA loss decreases from week 12 onwards to week 48 as shown by group C12 with 72% of patients loosing RNA at M3 (equaling week 12) and only 59% of patients at M12 (equaling week 48).

Another response typically induced by interferon-based treatments is normalization of ALT, 3 out of 7 patients already normalized ALT as a consequence of IFN treatment, however the other 4 patients failed to do so. One of these patients (01011) normalized ALT at the end of the E1 immunization series (normal ALT observed at week 29, weeks recalculated from onset of IFN based therapy), while another patient (01002) had a remarkable reduction in ALT level (Table 1). Also ALT normalization beyond week 24 of interferon/ribavirin treatment is rather exceptional as it was not noted in the study of Poynard et al. (1998, Table 2 therein) and only in a low number in the McHutchison et al. study (1998, Table 2 therein).

Thus in 3 out of 7 HCV infected patients, already on treatment for at least 10 weeks with interferon/ribavirin without loss of viral RNA, a late (later than week 12) viral or additional biochemical response was observed after E1 vaccination was initiated in addition to interferon/ribavirin treatment.

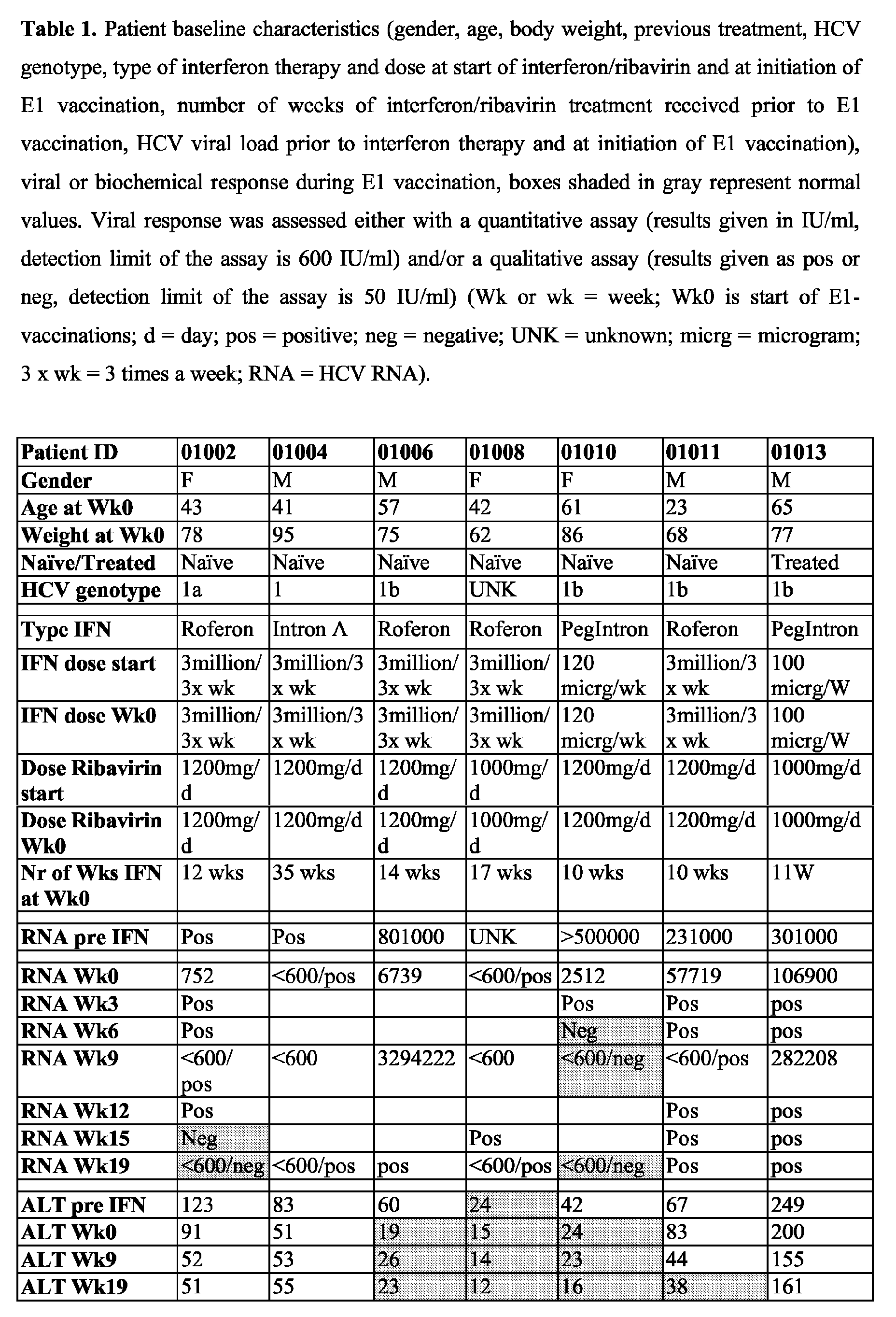

### EXAMPLE 2. Use of E1 prevents viral or biochemical breakthrough during IFN-treatment.

In a phase I/II, prospective, open label, single center, exploratory study, 5 patients who were HCV-RNA negative after at least 12 weeks of IFN treatment combined with ribavirin (see Table 2 for baseline characteristics and description of interferon/ribavirin therapy) were given a course of E1 vaccinations. This course consisted 6 intra-muscular immunizations with 20 µg E1 adsorbed on 0.13% of alum (the E1 protein was expressed in and purified from mammalian cells and further converted into oligomeric particles or viral-like particles as described in Example 19 of PCT/EP02/14480) administered with a 3 week interval, while the interferon-ribavirin treatment was continued. Both the viral and biochemical responses were analyzed prior to, during and after the E1 vaccination series. Viral response was measured with quantitative (Amplicor HCV Monitor 2.0, Roche, BranchBurg, NJ, USA), and/or qualitative methods (Amplicor HCV Amplification kit 2.0/Cobas Amplicon Detection kit 2.0, Roche). Biochemical response was assessed by measuring ALT using standard methods.

Remarkably, none of these patients experienced a viral or biochemical breakthrough (Table 2), in fact one of the patients even normalized ALT during the E1 vaccination series (patient 01012, see Table 2). Although the number of patients in this study is small, viral breakthrough could have been expected as it may occur as frequently as 28% as shown for genotype 1b infected, treatment naive, persons between month 3 and 12 (Table 2, group C12 in Pol et al. 2000).

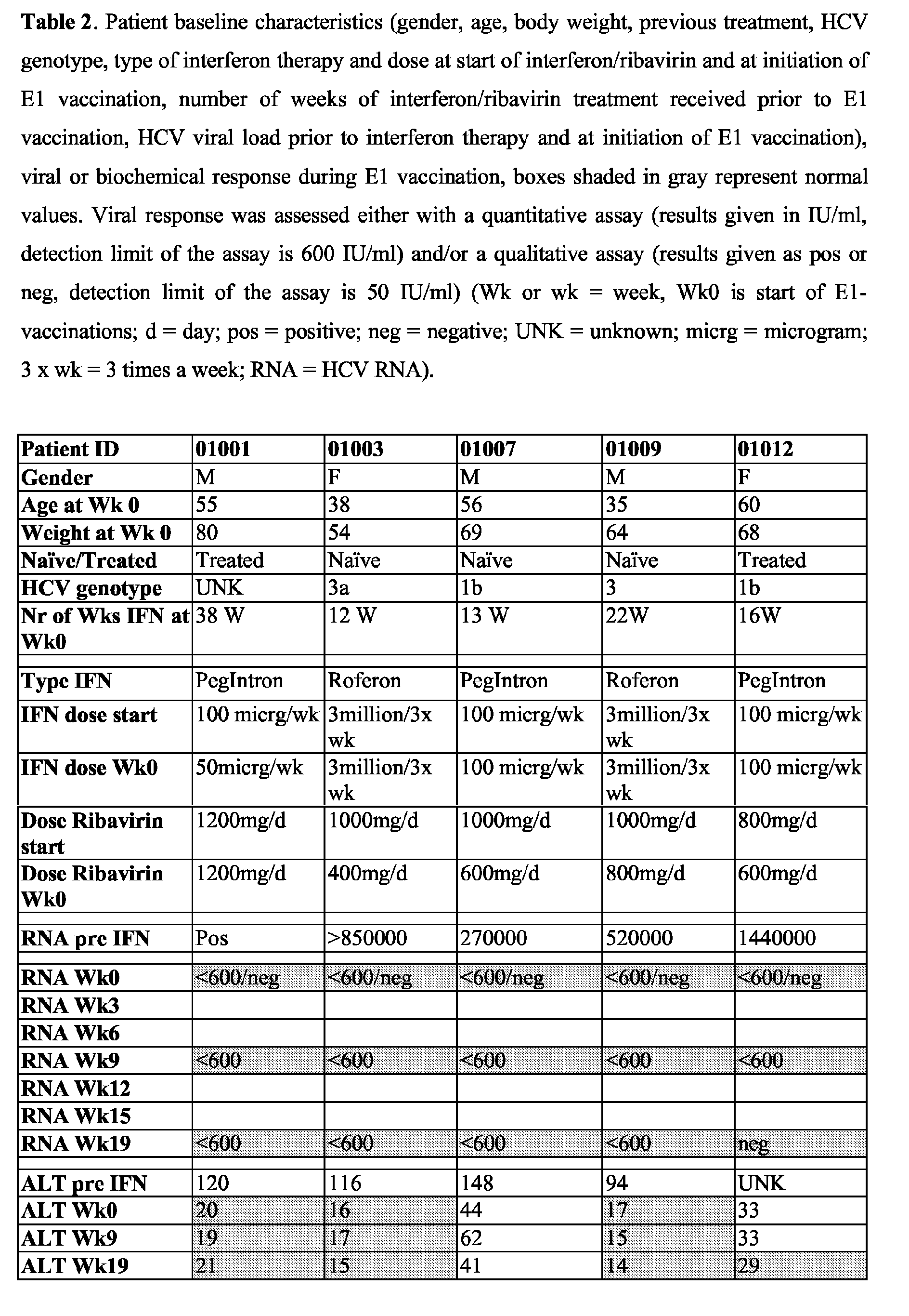

### EXAMPLE 3. Use of E1 to prevent viral or biochemical relapse induced by cessation of IFN-ribavirin treatment.

A total of 12 patients (a combination of the patients of Examples 1 and 2) were treated with interferon-ribavirin for at least 10 weeks after which E1 vaccination was initiated while interferon-ribavirin treatment continued. Seven patients experienced a viral response at the end of the E1-vaccine/interferon treatment period. Two of these patients did not yet have such a response at week 12 (Example 1) and there were no breakthrough infections reported (Example 2). It should be noted that two of the patients without detectable virus at the end of E1 vaccine/interferon treatment were non-responders to previous treatment (patient 01001 and 01012). Thus, the combined observations of Examples 1 and 2 lead to the conclusion that E1 vaccination may have changed the slope of viral load response in the second phase (beyond week 12) which may lead to an improved sustained viral response rate. Consequently these patients are followed up for another 6 months after cessation of the interferon treatment. At that time point their viremia is analyzed again.
The number of relapses (patients with negative RNA at the end of interferon treatment but RNA positive again 6 months later) is compared with published data. Results of published studies indicated a relapse rate of 15-19% (Fried et al. 2002, Figure 1 therein comparing end-of treatment response rate with sustained response rate for both groups receiving either type of interferon in combination with ribavirin); 13-18% (Manns et al. 2001, Table 2 therein comparing end-of treatment response rate with sustained response rate for the three groups receiving either type of interferon in combination with ribavirin); 19% (Poynard et al. 1998, Table 2 therein for the group receiving interferon in combination with ribavirin for 48 weeks); 24% (McHutchison et al. 1998, Table 2 therein for the group receiving interferon in combination with ribavirin for 48 weeks); or 23% for a genotype 1b infected patient study (Table 2, group C12 in Pol et al. 2000). For patients that have been treated before the relapse rate may be even as high as 50% or higher (Shiffman et al. 2002).

### EXAMPLE 4. Use of E1 to increase efficacy of IFN-ribavirin based therapies in patients infected with genotype 1.

A total of 6 treatment-naïve patients, infected with genotype 1 were treated with a combination of interferon-ribavirin for at least 10 weeks after which E1 vaccination was initiated while interferon-ribavirin treatment continued. The baseline characteristics and description of interferon/ribavirin therapy of these patients is summarized in Table 3. The E1 vaccination consisted out of a series of 6 intra-muscular immunizations with 20 µg E1 adsorbed on 0.13% of alum (the E1 protein was expressed in and purified from mammalian cells and further converted into oligomeric particles or viral-like particles as described in Example 19 of PCT/EP02/14480). This therapeutic vaccine was administered in a 3-weekly regimen.

Both the viral response and biochemical response were analyzed prior, during and after E1 vaccination. Viral response was measured with quantitative (Amplicor HCV Monitor 2.0, Roche, BranchBurg, NJ, USA), and/or qualitative methods (Amplicor HCV Amplification kit 2.0/Cobas Amplicon Detection kit 2.0, Roche). Biochemical response was assessed by measuring ALT using standard methods. In this group only one patient had lost HCV-RNA (patient nr 01007) and two more patients (nrs 01006 and 01010) had normalized their ALT prior to initiation of E1 vaccination.

Remarkably, two more patients out of 6 resolved their infection after initiation of E1 vaccination, patient 01002 at week 27, and patient 01010 at week 16 (Table 3, weeks calculated from start of IFN-based therapy). Although the number of patients in this study is small this frequency of late RNA resolution both by quantitative as well as qualitative methods is high (33%) as compared to what is known from other studies without additional vaccination. Especially the case resolving HCV at week 27 is an unexpected finding since in several studies no increased numbers of loss of HCV-RNA were noted comparing groups receiving 24 or 48 weeks of interferon-ribavirin treatment (Poynard et al. 1998, Table 2 therein; McHutchison et al. 1998, Table 2 therein). On the contrary loss of HCV-RNA observed at wk 48 seem to be somewhat lower which may be a consequence of breakthrough infections occurring between week 24 and week 48. The studies of Poynard and McHutchison do not give data of frequency of loss of HCV-RNA at week 12. The study of Pol and colleagues (2000, Fig. 4 therein) in genotype 1b patients does however give these data and reveals that almost no overall increase in viral clearing is observed between week 12 and week 48 of treatment with interferon/ribavirin (comparing a frequency of 58% in group C4 at M3 (which equaling week 12) with a frequency of 59% in group C12 at M12 (which equaling week 48)). In addition, also in this study, the number of patients showing viral RNA loss decreases from week 12 onwards to week 48 as shown by group C12 with 72% of patients lost RNA at M3 (equaling week 12) and only 59% of patients at M12 (equaling week 48).

Another response typically induced by interferon based treatments is normalization of ALT. Two out of 6 patients already normalized ALT as a consequence of IFN treatment, however the other 3 patients failed to do so. One of these patients (01011) normalized ALT at the end of the E1 immunization series (normal ALT observed at week 29), while another patient (01002) had a remarkable reduction in ALT level (Table 3). Also ALT normalization beyond week 24 is rather exceptional as it was not noted in the study of Poynard et al. (1998, Table 2 therein) and only in a low number of patients in the McHutchison et al. study (1998, Table 2 therein).

Thus in 3 out of 6 genotype 1 infected patients, who were already treated for at least 10 weeks with interferon/ribavirin, a late (later than week 12) viral or biochemical response was observed after E1 vaccination was initiated in addition to interferon/ribavirin treatment.

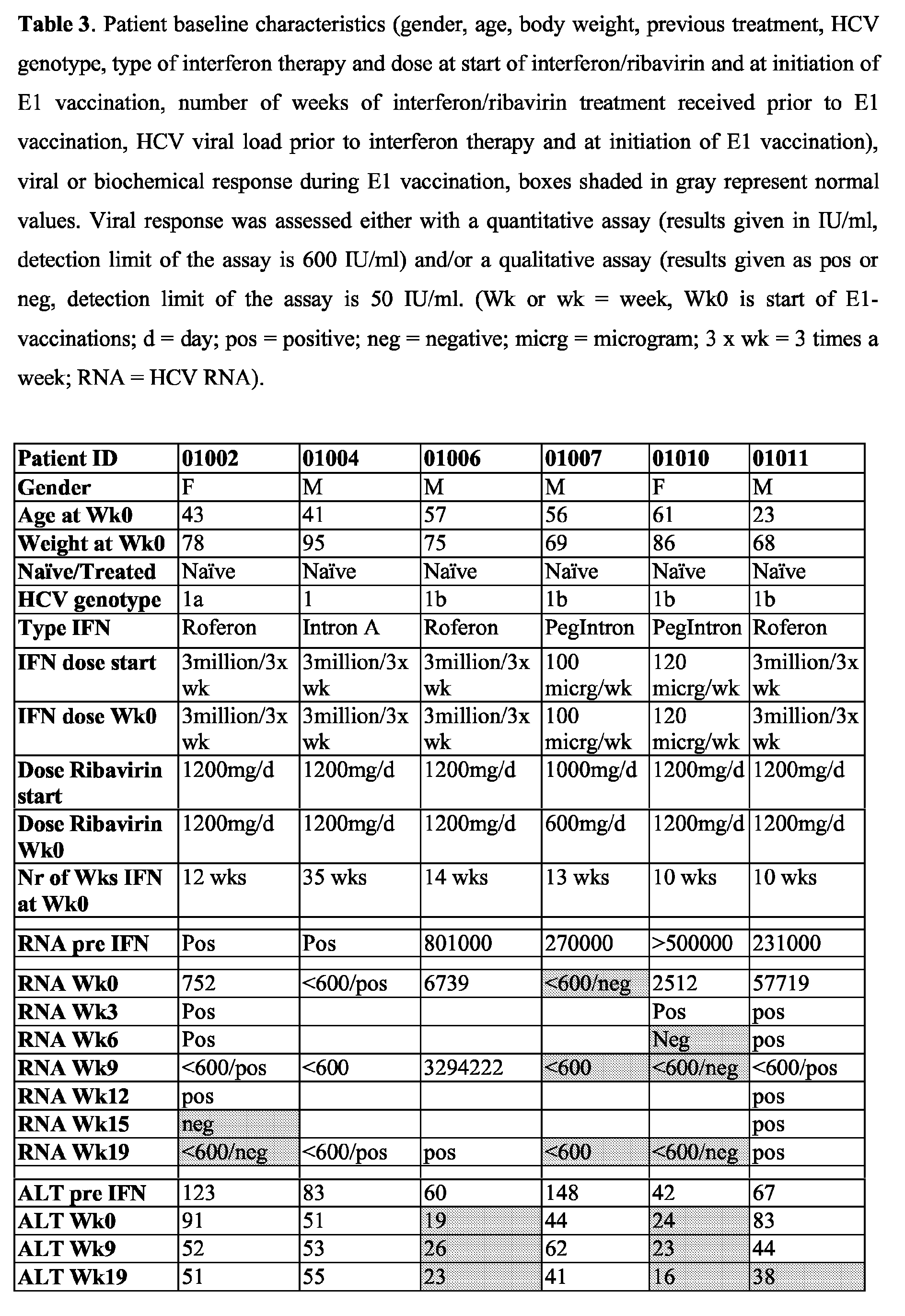

### EXAMPLE 5. Association of E1 specific vaccine-induced immunological response and increased efficacy of interferon-ribavirin therapy.

In patients 01002, 01010, 01011, and 01012, described in Examples 1 and 4, viral or biochemical response was only observed after initiation of E1 vaccination while IFN-ribavirin continued. The immune response to the vaccine was assessed by measuring the humoral response and/or the cellular response to the E1 protein, prior to and after the vaccination series. The results are presented in Table 4. Both patients 01002 and 01010 had rather low amounts (typically a median value of 100 mU is observed in a group of genotype 1 infected patients not receiving any treatment) of circulating antibodies against E1 prior to E1 vaccination, but the antibody level increased by a factor of 2 in patient 01002 and 15-fold in patient 01010. In addition, all 4 patients had prior to E1 vaccination a T-cell proliferation which is considered negative, but converted to a high level of E1-specific T-cell proliferation after E1 vaccination. This clearly demonstrates that E1-specific immune responses were associated with the late viral clearing or biochemical response in these patients.

On average, this study demonstrated that E1Ab levels could be increased from 98.7 to 530.4 mU/ml (median values) by means of therapeutic vaccination during interferon-ribavirin treatment. These levels were not significantly different from those observed in patients receiving E1 therapeutic vaccination monotherapy (see also example 6). Therefore this study shows for the first time that the side effects of interferon (neutropenia) and ribavirin (anemia) do not negatively influence to possibility to induce immune responses to therapeutic vaccination in this setting.

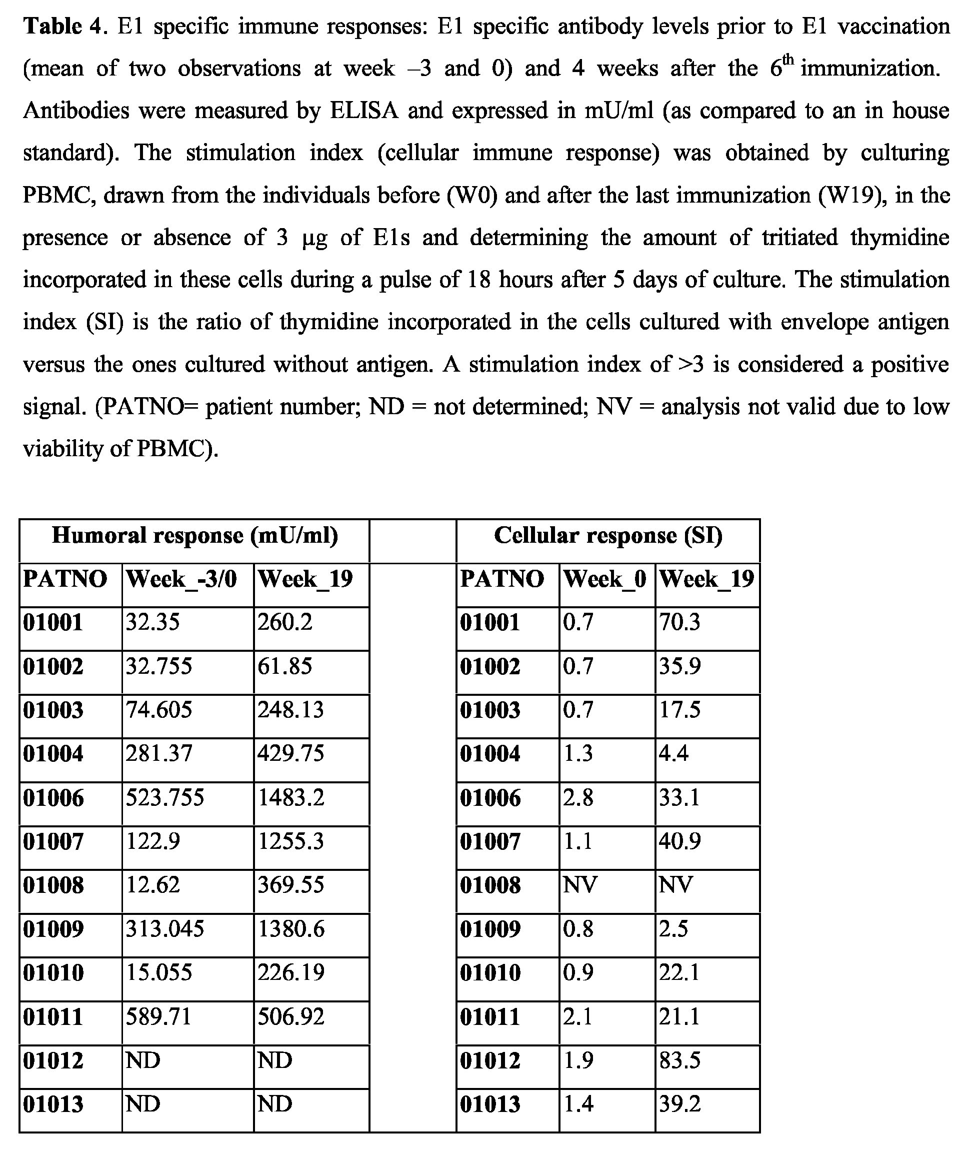

### EXAMPLE 6. Lack of interferon/ribavirin adjuvant like properties for E1 vaccination.

Several publications have stressed the adjuvant properties of interferon or ribavirin. If this would have been the case we have observed a higher level of antibodies in patients vaccinated with E1 while receiving interferon/ribavirin compared to patients receiving only the E1 vaccine. Consequently we compared the humoral responses induced in the patients of Examples 1 and 2 with a group of 9 patients that also received 6 E1 immunizations of 20 µg with a 3-week interval. The immune response to the vaccine was assessed by measuring the humoral response to the E1 protein, prior to and after the vaccination series.

As can be judged from Table 5, the baseline median value of the E1 antibody level is very similar between both groups, there is however a somewhat higher variability in the group receiving interferon compared to the group without interferon as can be judged from the Q1 (p25 percentile) and Q3 (p75 percentile) levels. Four weeks after the 6^{th} immunization, the increase in antibody level is very similar. As this is true for the median and also for the p25 and p75 percentile, these data clearly indicate that the simultaneous administration of systemic interferon and ribavirin did **not** have an adjuvant type of effect on the E1-induced responses.

**Table 5.**

| E1 specific antibody levels prior to E1 vaccination (mean of two observations at week -3 and 0 for the study in which patients received also interferon and weeks 48 and 50 for those patients who received E1 vaccination as monotherapy) and 4 weeks after the 6^{th} immunization. Antibodies were measured by ELISA and expressed in mU/ml (as compared to an in house standard). (PATNO = patient number; ND = not determined) | | | | | | | | |
|---|---|---|---|---|---|---|---|---|
| **Patients receiving interferon** | | | | | **Patients without interferon** | | | |
| **PATNO 908/** | **Wk -3/0** | **Wk19** | **Fold increase** | | **PATNO 913/** | **Wk48/50** | **Wk69** | **Fold increase** |
| **01001** | 32.35 | 260.2 | | | **01006** | 85.675 | 530.4 | |
| **01002** | 32.755 | 61.85 | | | **01010** | 1352.3 | 1641.6 | |
| **01003** | 74.605 | 248.13 | | | **02002** | 105.35 | 855.2 | |
| **01004** | 281.37 | 429.75 | | | **03011** | 137.55 | 670.4 | |
| **01006** | 523.755 | 1483.2 | | | **03013** | 308.65 | 434.8 | |
| **01007** | 122.9 | 1255.3 | | | **03018** | 0 | 131.1 | |
| **01008** | 12.62 | 369.55 | | | **03026** | 131.8 | 474 | |
| **01009** | 313.045 | 1380.6 | | | **05001** | 35.995 | 208 | |
| **01010** | 15.055 | 226.19 | | | **05006** | 61.725 | 701.1 | |
| **01011** | 589.71 | 506.92 | | | | | | |
| **01012** | ND | ND | | | | | | |
| **median** | **98.7525** | **399.65** | **4.04** | | **Median** | **105.35** | **530.4** | **5.03** |
| **Q1** | **32.4513** | **251.148** | **7.74** | | **Q1** | **61.725** | **434.8** | **7.04** |
| **Q3** | **305.126** | **1068.21** | **3.50** | | **Q3** | **137.55** | **701.1** | **5.10** |

### EXAMPLE 7. E1 detected as an immune correlate of response to antiviral treatment

In a phase I/II, prospective, open label, single center, exploratory study, 10 healthy male volunteers (group A), 3 men who had cleared HCV infection after interferon-based treatment (group B), and 8 patients (3 men and 5 women) suffering from therapy-resistant chronic HCV (group C) were given sub-epidermal doses of non-adjuvanted E1 (4 µg E1 in 0.1 ml PBS per dose) at weeks 0,4, and 8. Safety evaluations were performed after each dose. The humoral immune response to E1 was assessed at weeks -3, 0, 4, 8, 10, and 12 by measuring the serum levels of anti-E1 Ab. The E1-specific cellular immune responses were monitored at weeks -3 and 10 by measuring the in vitro proliferative responses of PBMC stimulated with 0.03, 0.3, and 3 µg of E1. These lympoproliferative responses were measured by ³H-thymidine incorporation and expressed as stimulation indices (SI) with a SI >= 3 considered significant.

Local reactions at the injection site were seen in 14 of the 21 participants. Four out of 8 group C patients and 1 group B volunteer reported transient flu-like symptoms after injection. No other noteworthy side effects were observed. In group A, all 10 volunteers were negative for anti-E1 antibodies prior to vaccination and only three mounted a detectable anti-E1 antibody response by week 10. The subject with the highest anti-E1 titer (1203 mU/ml at week 10) was the only one with a SI>=3 at week 10. In group B one subject had low-titer anti-E1 prior to vaccination. This subject mounted the highest anti-E1 response at week 10 (251 mU/ml) and also had a SI >=3 at week 10. The other two subjects mounted an early anti-E1 antibody response after two vaccine doses but displayed no cellular response. In group C 7 out of 8 patients had anti-E1 antibodies at baseline. At week 10 a rise in titer was observed in only 2 subjects. An E1-specific lymphoproliferative response was measured at week 10 in the patient with the highest antibody response (1058 mU/ml).

In three subjects who had previously cleared HCV infection after antiviral treatment, subepidermal administration of a low dose of E1 induced rapid and clear anamnestic responses. These data demonstrate that E1-specific immune responses may be induced during resolving HCV infections and that memory (B and T) cells can be restimulated with suboptimal doses of E1 antigen in such subjects. It may therefore be possible that such E1-specific immune responses contribute to the clearance of HCV infection when present before, during or after interferon therapy.

### REFERENCES

1. Alpar,H.O. & Bramwell,V.W. Current status of DNA vaccines and their route of administration. *Crit Rev. Ther. Drug Carrier Syst.* **19**, 307-3 83 (2002).
2. Barbaro,G. & Barbarini,G. Consensus interferon for chronic hepatitis C patients with genotype 1 who failed to respond to, or relapsed after, interferon alpha-2b and ribavirin in combination: an Italian pilot study. *Eur. J Gastroenterol. Hepatol.* **14,** 477-483 (2002).
3. Barth,H. *et al.* Cellular binding of HCV envelope glycoprotein E2 requires cells surface heparan sulfate proteoglycans. Hepatology 36, 214A. 2002.
4. Barton,G.M. & Medzhitov,R. Toll-like receptors and their ligands. *Curr. Top. Microbiol. Immunol.* **270,** 81-92 (2002).
5. Byl,B. *et al.* OM197-MP-AC induces the maturation of human dendritic cells and promotes a primary T cell response. *Int Immunopharmacol.* **3**, 417-425 (2003).
6. Choo,Q.L. *et al.* Vaccination of chimpanzees against infection by the hepatitis C virus. *Proc. Natl. Acad. Sci. U. S. A* **91,** 1294-1298 (1994).
7. Chutaputti,A. Adverse effects and other safety aspects of the hepatitis C antivirals. *J Gastroenterol. Hepatol.* **15 Suppl,** E156-E163 (2000).
8. da Silva,L.C. *et al.* High rate of sustained response to consensus interferon plus ribavirin in chronic hepatitis C patients resistant to alpha-interferon and ribavirin: a pilot study. *J Gastroenterol.* **37,** 732-736 (2002).
9. Di Bisceglie,A.M. *et al.* Ribavirin as therapy for chronic hepatitis C. A randomized, double-blind, placebo-controlled trial. *Ann. Intern. Med.* **123**, 897-903 (1995).
10. Di Bisceglie,A.M., McHutchison,J. & Rice,C.M. New therapeutic strategies for hepatitis *C. Hepatology* **35,** 224-231 (2002).
11. Dockrell,D.H. & Kinghorn,G.R. Imiquimod and resiquimod as novel immunomodulators. *J Antimicrob. Chemother.* **48,** 751-755 (2001).
12. Dusheiko,G. *et al.* Ribavirin treatment for patients with chronic hepatitis C: results of a placebo-controlled study. *J Hepatol.* **25**, 591-598 (1996).
13. Falck-Ytter,Y. *et al.* Surprisingly small effect of antiviral treatment in patients with hepatitis *C. Ann. Intern. Med.* **136,** 288-292 (2002).
14. Felipe,M. *et al.* A prospective and randomized study using ribavirin as monotherapy for the treatment of naive patients with chronic hepatitis C. *Braz. J Infect. Dis.* **4,** 183-191 (2000).
15. Forns,X. *et al.* DNA immunization of mice and macaques with plasmids encoding hepatitis C virus envelope E2 protein expressed intracellularly and on the cell surface. *Vaccine* **17,** 1992-2002 (1999).
16. Forns,X. *et al.* Vaccination of chimpanzees with plasmid DNA encoding the hepatitis C virus (HCV) envelope E2 protein modified the infection after challenge with homologous monoclonal HCV. *Hepatology* **32**, 618-625 (2000).
17. Fried,M.W. *et al.* Therapy of chronic hepatitis B with a 6-month course of ribavirin. *J Hepatol.* **21,** 145-150 (1994).
18. Fried,M.W. *et al.* Peginterferon alfa-2a plus ribavirin for chronic hepatitis C virus infection. *N. Engl J Med.* **347,** 975-982 (2002).
19. Fujihashi,K. *et al.* Cytokine-specific ELISPOT assay. Single cell analysis of IL-2, IL-4 and IL-6 producing cells. *J*. *Immunol. Methods* **160,** 181-189 (1993).
20. Galban-Garcia,E. *et al.* Efficacy of ribavirin in patients with chronic hepatitis B. *J Gastroenterol.* **35,** 347-352 (2000).
21. Grakoui,A., Wychowski,C., Lin,C., Feinstone,S.M. & Rice,C.M. Expression and identification of hepatitis C virus polyprotein cleavage products. *J. Virol.* **67**, 1385-1395 (1993).
22. Hemmi,H. *et al.* Small anti-viral compounds activate immune cells via the TLR7 MyD88-dependent signaling pathway. *Nat. Immunol.* **3**, 196-200 (2002).
23. Houghton,M. *et al.* Prospects for prophylactic and therapeutic hepatitis C virus vaccines. *Princess Takamatsu Symp.* **25**, 237-243 (1995).
24. Hu,G.J., Wang,R.Y., Han,D.S., Alter,H.J. & Shih,J.W. Characterization of the humoral and cellular immune responses against hepatitis C virus core induced by DNA-based immunization. *Vaccine* **17**, 3160-3170 (1999).
25. Ito,T., Amakawa,R. & Fukuhara,S. Roles of toll-like receptors in natural interferon-producing cells as sensors in immune surveillance. *Hum. Immunol.* **63,** 1120-1125 (2002).
26. Johnson,D.A. *et al.* Synthesis and biological evaluation of a new class of vaccine adjuvants: aminoalkyl glucosaminide 4-phosphates (AGPs). *Bioorg. Med. Chem Lett* ***9,*** 2273-2278 (1999).
27. Lai,M.Y. *et al.* Long-term efficacy of ribavirin plus interferon alfa in the treatment of chronic hepatitis C. *Gastroenterology* **111,** 1307-1312 (1996).
28. Lauer,G.M. & Walker,B.D. Hepatitis C virus infection. *N. Engl J. Med.* **345,** 41-52 (2001).
29. Lee,J. *et al.* Molecular basis for the immunostimulatory activity of guanine nucleoside analogs: Activation of Toll-like receptor 7. *Proc. Natl. Acad. Sci. U. S. A* **100**, 6646-6651 (2003).
30. Lopez-Dias de Cerio AL *et al.* T(h)1 but not T(h)0 cell help is efficient to induce cytotoxic T lymphocytes by immunization with short synthetic peptides. *Int Immunol.* **11**, 2025-2034 (1999).
31. Manns,M.P. *et al.* Peginterferon alfa-2b plus ribavirin compared with interferon alfa-2b plus ribavirin for initial treatment of chronic hepatitis C: a randomised trial. *Lancet* **358**, 958-965 (2001).
32. McHutchison,J.G. *et al.* Interferon alfa-2b alone or in combination with ribavirin as initial treatment for chronic hepatitis C. Hepatitis Interventional Therapy Group. *N. Engl J Med.* **339**, 1485-1492 (1998).
33. Nevens,F. *et al.* Antifibrotic activity correlates with immune response to E1 therapeutic vaccination in 24 patients with chronic active hepatitis C. J Hepatol. 38 (Suppl. 2), 17. 2003.
34. Persing,D. *et al.* Taking toll: lipid A mimetics as adjuvants and immunomodulators. *Trends Microbiol.* **10,** S32 (2002).
35. Pockros,P.J. *et al.* The safety and tolerability of daily infergen plus ribavirin in the treatment of naiive chronic hepatitis C patients. *J Viral Hepat.* **10**, 55-60 (2003).
36. Pohlmann,S. *et al.* Hepatitis C virus glycoproteins interact with DC-SIGN and DC-SIGNR. *J Virol.* **77**, 4070-4080 (2003).
37. Pol,S. *et al.* Combination of ribavirin and interferon-alfa surpasses high doses of interferon-alfa alone in patients with genotype-1b-related chronic hepatitis C. *Hepatology* **31**, 1338-1344 (2000).
38. Poynard,T. *et al.* Randomised trial of interferon alpha2b plus ribavirin for 48 weeks or for 24 weeks versus interferon alpha2b plus placebo for 48 weeks for treatment of chronic infection with hepatitis C virus. International Hepatitis Interventional Therapy Group (IHIT). *Lancet* **352,** 1426-1432 (1998).
39. Reichard,O. *et al.* Randomised, double-blind, placebo-controlled trial of interferon alpha-2b with and without ribavirin for chronic hepatitis C. The Swedish Study Group. *Lancet* **351,** 83-87 (1998).
40. Rein,A. *et al.* Inactivation of murine leukemia virus by compounds that react with the zinc finger in the viral nucleocapsid protein. *J. Virol.* **70,** 4966-4972 (1996).
41. Riedl,P., Buschle,M., Reimann,J. & Schirmbeck,R. Binding immune-stimulating oligonucleotides to cationic peptides from viral core antigen enhances their potency as adjuvants. *Eur. J. Immunol.* **32,** 1709-1716 (2002).
42. Scarselli,E. *et al.* The human scavenger receptor class B type I is a novel candidate receptor for the hepatitis C virus. *EMBO J* **21,** 5017-5025 (2002).
43. Sheppard,P. *et al.* IL-28, IL-29 and their class II cytokine receptor IL-28R. *Nat. Immunol.* **4**, 63-68 (2003).
44. Shiffinan,M.L. Improvement in liver histopathology associated with interferon therapy in patients with chronic hepatitis C. *Viral Hepatitis Reviews* **5**, 27-43 (1999).
45. Shiffman,M. Retreatment of HCV non-responders with peginterferon and ribavirin: results from the lead-in phase of the hepatitis C antiviral long-term treatment against cirrhosis (halt-C) trial. *Hepatology* **36**, (2002).
46. Shimotohno,K. *et al.* Processing of the hepatitis C virus precursor protein. *J. Hepatol.* **22**, 87-92 (1995).
47. Shirai,M. *et al.* Use of intrinsic and extrinsic helper epitopes for in vivo induction of anti-hepatitis C virus cytotoxic T lymphocytes (CTL) with CTL epitope peptide vaccines. *J. Infect. Dis.* **173,** 24-31 (1996).
48. Song,M.K., Lee,S.W., Suh,Y.S., Lee,K.J. & Sung,Y.C. Enhancement of immunoglobulin G2a and cytotoxic T-lymphocyte responses by a booster immunization with recombinant hepatitis C virus E2 protein in E2 DNA-primed mice. *J*. *virol.* **74**, 2920-2925 (2000).
49. Sorbera,L.A., Silvestre,J.S., Castaner,J. & Martin,L. VX-497. Anti-HCV, Antipsoriatic, IMPDH inhibitor. Drugs of the Future 25, 809-814. 2000.
50. Tan,S.L., Pause,A., Shi,Y. & Sonenberg,N. Hepatitis C therapeutics: current status and emerging strategies. *Nat. Rev. Drug Discov.* **1**, 867-881 (2002).
51. Uno-Furuta,S. *et al.* Induction of virus-specific cytotoxic T lymphocytes by in vivo electric administration of peptides. *Vaccine* **19**, 2190-2196 (2001).
52. Vilcek,J. Novel interferons. *Nat. Immunol.* **4,** 8-9 (2003).
53. Walewski,J.L., Keller,T.R., Stump,D.D. & Branch,A.D. Evidence for a new hepatitis C virus antigen encoded in an overlapping reading frame. *RNA.* **7**, 710-721 (2001).
54. Walker,M.P., Appleby,T.C., Zhong,W., Lau,J.Y. & Hong,Z. Hepatitis C virus therapies: current treatments, targets and future perspectives. *Antivir. Chem Chemother.* **14,** 1-21 (2003).
55. Watson,J. Prospects for hepatitis C virus therapeutics: levovirin and viramidine as improved derivatives of ribavirin. *Curr. Opin. Investig. Drugs* **3**, 680-683 (2002).
56. Xu,Z. *et al.* Synthesis of a novel hepatitis C virus protein by ribosomal frameshift. *EMBO J.* **20**, 3 840-3 848 (2001).

## Claims

1. Use of an isolated HCV E1 protein and/or of an HCV E1 DNA vector for the manufacture of an HCV vaccine for therapeutic treatment of an HCV-infected mammal by a method comprising administering said HCV vaccine before and/or during and/or after a therapy comprising at least one antiviral agent.

2. Use of at least one antiviral for the manufacture of a pharmaceutical composition for therapeutic treatment of an HCV-infected mammal by a method comprising administering said pharmaceutical composition before and/or during and/or after a therapy comprising administering an HCV vaccine wherein said HCV vaccine is at least comprising an isolated HCV E1 protein and/or an HCV E1 DNA vector as active substance.

3. The use according to claim 1 or 2 wherein said therapeutic treatment induces an immune response in said HCV-infected mammal.

4. The use according to claim 1 or 2 wherein said therapeutic treatment enhances HCV viral clearance in said HCV-infected mammal.

5. The use according to claim 1 or 2 wherein said therapeutic treatment suppresses HCV viral breakthrough in said HCV-infected mammal.

6. The use according to claim 1 or 2 wherein HCV viral rebound is suppressed after cessation of said therapeutic treatment.

7. The use according to any of claims 1 or 6 wherein said at least one antiviral agent is an interferon or a variant or consensus sequence thereof, or an inducer of interferon.

8. The use according to claim 7 wherein said interferon is a type I or a type III interferon or a variant or consensus sequence of any thereof.

9. The use according to claim 8 wherein said type I interferon is chosen from an interferon-alfa, interferon-beta, interferon-omega or interferon-tau or wherein said type III interferon is chosen from IL-28A, IL-28B or IL-29.

10. The use according to claim 7 wherein said inducer of interferon is chosen from TLR7-ligands, TLR-9 ligands, ANA245, or ANA971.

11. The use according to any of claims 1 to 6 wherein if said at least one antiviral agent is interferon or a variant or consensus sequence thereof, said method further comprises administering an interferon-enhancer or at least one additional antiviral agent.

12. The use according to claim 11 wherein said interferon-enhancer is chosen from interferon gamma, ribavirin, levovirin, viramidine, amantadine, thymosin alfa-1, histamine dihydrochloride, a methyldonor, or ANA246.

13. The use according to any of claims 1 to 6 or 11 wherein said at least one antiviral agent or said at least one additional antiviral agent is chosen from an IMPDH-inhibitor, an anti-HCV antibody, an immune modulator, a HCV-receptor inhibitor, an HCV fusion inhibitor, a modulator of host cell function required for HCV replication, a molecule targeting a step in the HCV life cycle, a molecule targeting an HCV IRES, a molecule binding to HCV RNA, a molecule targeting an HCV NS2-NS3 protease, a molecule targeting an HCV NS3 protease, a molecule targeting an HCV NS3 RNA helicase, a molecule targeting an HCV NS3-NS4A protease, a molecule targeting an HCV NS5B RNA-dependent RNA polymerase, and a molecule inhibiting binding of HCV E2 or HCV NS5 to protein kinase R, a molecule targeting an HCV Core protein, a molecule targeting an HCV E1 protein or a molecule targeting an HCV E2 protein.

14. The use according to claim 13 wherein said IMPDH-inhibitor is chosen from ribavirin or a variant thereof, viramidine, mizoribine monophosphate, merimepodib or mycophenolic acid or a variant thereof.

15. The use according to any of claims 11 or 13 wherein if said interferon-enhancer or IMPDH-inhibitor is ribavirin or a variant thereof an ameliorant of ribavirin-induced anemia is administered to the HCV-infected mammal.

16. The use according to claim 15 wherein said ameliorant of ribavirin-induced anemia is an antioxidant or erythropoietin.

17. The use according to any of claims 1 to 16 wherein said HCV E1 protein is an E1s protein or wherein said HCV E1 DNA vector is encoding an E1s protein.

18. The use according to any of claims 1 to 16 wherein said HCV E1 protein is defined by SEQ ID NO:1 or wherein said HCV E1 DNA vector is encoding an E1 protein defined by SEQ ID NO:1.

19. A pharmaceutical pack or kit at least comprising one or more containers with an HCV vaccine and one or more containers with at least one antiviral agent and wherein said HCV vaccine is at least comprising an isolated HCV E1 protein and/or an HCV E1 DNA vector as active substance.

20. The pharmaceutical pack or kit according to claim 19 wherein said at least one antiviral agent is an interferon or a variant or consensus sequence thereof or an inducer of interferon.

21. The pharmaceutical pack or kit according to claim 19 wherein said HCV E1 protein is an E1s protein or wherein said HCV E1 DNA vector is encoding an E1s protein.

22. The pharmaceutical pack or kit according to claim 21 wherein said HCV E1 protein is defined by SEQ ID NO:1 or wherein said HCV E1 DNA vector is encoding an E1 protein defined by SEQ ID NO:1.

23. The use according to any of claims 1 to 18 or the pharmaceutical pack or kit according to any of claims 19 to 22 wherein said HCV E1 protein is added to said HCV vaccine as a viral-like particle.

24. The use according to any of claims 1 to 18 or the pharmaceutical pack or kit according to any of claims 19 to 22 wherein the cysteine-thiol groups in said HCV E1 protein are reversibly or irreversibly blocked.


### Annex to the application documents-subsequently filed sequences listing

SEQUENCE LISTING

<110>  Innogenetics N.V.

<120>  HCV combination therapy

<130>  155

<160>  2

<170>  PatentIn version 3.1

<210>  1
<211>  135
<212>  PRT
<213>  hepatitis C virus

<400>  1

Tyr Glu Val Arg Asn Val Ser Gly Met Tyr His Val Thr Asn Asp Cys
1               5                   10                  15

Ser Asn Ser Ser Ile Val Tyr Glu Ala Ala Asp Met Ile Met His Thr
            20                  25                  30

Pro Gly Cys Val Pro Cys Val Arg Glu Asn Asn Ser Ser Arg Cys Trp
        35                  40                  45

Val Ala Leu Thr Pro Thr Leu Ala Ala Arg Asn Ala Ser Val Pro Thr
    50                  55                  60

Thr Thr Ile Arg Arg His Val Asp Leu Leu Val Gly Ala Ala Ala Phe
65                  70                  75                  80

Cys Ser Ala Met Tyr Val Gly Asp Leu Cys Gly Ser Val Phe Leu Val
                85                  90                  95

Ser Gln Leu Phe Thr Ile Ser Pro Arg Arg His Glu Thr Val Gln Asp
            100                 105                 110

Cys Asn Cys Ser Ile Tyr Pro Gly His Ile Thr Gly His Arg Met Ala
        115                 120                 125

Trp Asp Met Met Met Asn Trp
    130                 135

<210>  2

<211>  192

<212>  PRT

<213>  hepatitis C virus

<400>  2

Tyr Glu Val Arg Asn Val Ser Gly Met Tyr His Val Thr Asn Asp Cys
1               5                   10                  15

Ser Asn Ser Ser Ile Val Tyr Glu Ala Ala Asp Met Ile Met His Thr
            20                  25                  30

Pro Gly Cys Val Pro Cys Val Arg Glu Asn Asn Ser Ser Arg Cys Trp
        35                  40                  45

Val Ala Leu Thr Pro Thr Leu Ala Ala Arg Asn Ala Ser Val Pro Thr
    50                  55                  60

Thr Thr Ile Arg Arg His Val Asp Leu Leu Val Gly Ala Ala Ala Phe
65                  70                  75                  80

Cys Ser Ala Met Tyr Val Gly Asp Leu Cys Gly Ser Val Phe Leu Val
                85                  90                  95

Ser Gln Leu Phe Thr Ile Ser Pro Arg Arg His Glu Thr Val Gln Asp
            100                 105                 110

Cys Asn Cys Ser Ile Tyr Pro Gly His Ile Thr Gly His Arg Met Ala
        115                 120                 125

Trp Asp Met Met Met Asn Trp Ser Pro Thr Thr Ala Leu Val Val Ser
    130                 135                 140

Gln Leu Leu Arg Ile Pro Gln Ala Val Val Asp Met Val Ala Gly Ala
145                 150                 155                 160

His Trp Gly Val Leu Ala Gly Leu Ala Tyr Tyr Ser Met Val Gly Asn
                165                 170                 175

Trp Ala Lys Val Leu Val Val Met Leu Leu Phe Ala Gly Val Asp Gly
            180                 185                 190